# DEMANDE DE BREVET EUROPEEN

(11) **EP 1 938 788 A1**
(43) Date de publication de la demande: **02.07.2008**
(21) Numéro de dépôt: 07122554.4
(22) Date de dépôt: 06.12.2007
(51) Int. Cl.: A61K 8/31, A61K 8/58, A61K 8/81, A61K 8/891, A61K 8/898, A61Q 1/02, A61Q 19/00

(54) **Composition cosmétique comprenant un dérivé de bis urée**

(30) Priorité: 29.12.2006 FR 0656062
(71) Demandeur: L'Oréal, 75008 Paris (FR)
(72) Inventeur: Feltin, Charlotte, 75017, PARIS (FR)
(74) Mandataire: Chantraine, Sylvie Hélène

(57) **Abrégé**

La présente invention a pour objet une composition cosmétique comprenant au moins une phase grasse liquide continue, comprenant au moins un composé de formule générale (I), ou l'un de ses sels et/ou isomères : 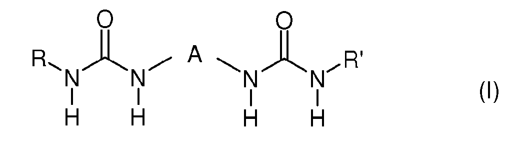 Et au moins une huile ayant un paramètre de solubilité 6. compris entre 0 et 7,00 (J/cm3) 1/2, et de préférence entre 0 et 5,00 (J/cm³)^{1/2}. L'invention concerne également un procédé de maquillage ou de soin des matières kératiniques comprenant l'application sur lesdites matières kératiniques d'une composition telle que décrite précédemment.

## Description

La présente invention se rapporte à une composition destinée au soin, ou au maquillage de la peau aussi bien du visage que du corps humain, des fibres kératiniques comme les cils, les sourcils, les cheveux ou encore des lèvres.

Cette composition peut être un fond de teint, un fard à joues ou à paupières, un anti-cernes, une crème à lèvres, un mascara, un produit de maquillage du corps, lorsqu'elle se présente sous forme colorée ou bien être une crème de soin pour la peau, un après-shampooing, un shampooing, une crème de protection solaire ou de coloration de la peau ou encore une pommade dermatologique, lorsqu'elle se présente sous forme non colorée.

Les compositions de soin ou de maquillage peuvent présenter des textures variées allant du fluide au solide. Une des difficultés rencontrées par les utilisatrices est de pouvoir bien étaler uniformément la composition sur toute la surface du visage ou du corps de manière à répartir uniformément le produit. Les compositions de textures épaisses ou solides sont difficiles à étaler en raison de leurs viscosités élevées. Les compositions de textures fluides ne sont pas toujours appropriées pour obtenir un maquillage uniforme, ne laissant pas de marques visibles sur la peau, notamment en raison de leur mauvais étalement sur toute la surface du visage à maquiller. Les textures crémeuses sont donc souvent recherchées.
Ces compositions crémeuses contiennent généralement un agent épaississant facilitant la prise de produit hors du conditionnement, sans perte de produit, permettant de répartir de façon homogène le produit sur la zone à traiter ou de prélever les quantités suffisantes de produit, pour obtenir l'effet cosmétique recherché. Lorsque la composition est suffisamment épaisse, une prise de produit au doigt fait apparaître, à la surface du produit dans le conditionnement, des parties en creux et lors de la prise suivante, la surface de produit est restée en l'état, notamment lors de la fermeture précédente du pot ; la crème paraît alors polluée, provoquant une insatisfaction de la part des consommateurs.

Le non nivellement en surface de la crème, après chaque utilisation, apparaît en particulier pour une crème de texture riche et/ou épaissie, ne s'écoulant pas.

Il subsiste donc le besoin d'une composition de maquillage, de traitement ou de soin de la peau et des fibres kératiniques présentant un bon nivellement de la surface dans son conditionnement, donnant l'impression d'ouvrir à chaque fois un nouveau pot, et de toucher un produit non pollué.

Les inventeurs ont découvert qu'une telle composition est obtenue en associant dans la composition au moins un dérivé de bis-urée et au moins une huile particulière.

La présente invention a pour objet, selon un premier aspect, une composition cosmétique comprenant au moins une phase grasse liquide continue, comprenant au moins un composé de formule générale (1), ou l'un de ses sels et/ou isomères : 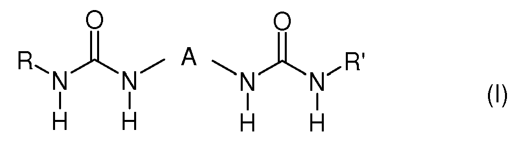 dans laquelle :
- A est un groupement de formule (II) : 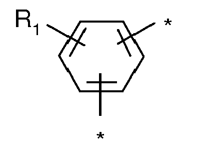 avec R1 étant un radical alkyle C₁ à C₄ linéaire ou ramifié, et les * symbolisant les points de rattachement du groupement A à chacun des deux atomes d'azote du reste du composé de formule générale (1), et
- R et R', identiques ou différents, sont choisis parmi :
- i) les radicaux de formule (III) : 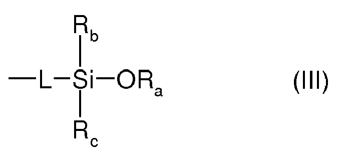
dans laquelle :
- L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S;
- Ra est :
   a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
   b) un radical siliconé de formule : 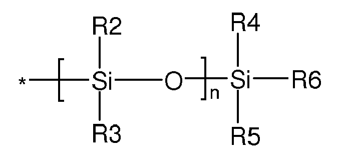 avec n étant compris entre 0 et 100, notamment entre 1 et 80, voire 2 à 20;
      et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;
- Rb et Rc sont, indépendamment l'un de l'autre, choisis parmi:
   a) les radicaux carbonés, notamment hydrocarbonés (alkyles), linéaires, ramifiés et/ou cycliques, saturés
      ou insaturés, comprenant 1 à 18 atomes de carbone, et pouvant comprendre 1 à 4 hétéroatomes choisis parmi N, O, Si et S;
   b) les radicaux de formule : 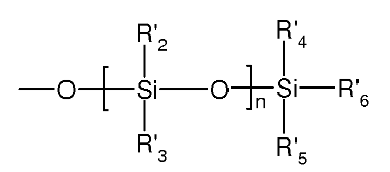 avec n étant compris entre 0 et 100, notamment entre 1 et 80, voire 2 à 20;
      et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
      et
- ii) les radicaux alkyle en C₁ à C₃₀, linéaire, ramifié et/ou cyclique, saturé ou insaturé, et comprenant éventuellement 1 à 3 hétéroatomes choisis parmi O, S, F et N;
étant entendu que l'un au moins des radicaux R et/ou R' est de formule (III) ; et
au moins une huile ayant un paramètre de solubilité δₐ compris entre 0 et 7,00 (J/cm³)^{1/2}, et de préférence entre 0 et 5,00 (J/cm³)^{1/2}.s

Selon un second aspect, l'invention a pour objet un procédé de maquillage ou de soin des matières kératiniques comprenant l'application sur lesdites matières kératiniques d'au moins une composition telle que définie précédemment.

Selon un troisième aspect, l'invention a pour objet l'utilisation d'une composition telle que définie précédemment pour obtenir un résultat maquillage lissant par une nivellement du relief de la peau et/ou mat et/ou doux au toucher

### Bis-urées siliconées

Les composés de type bis-urée selon l'invention répondent à la formule générale (1) suivante : 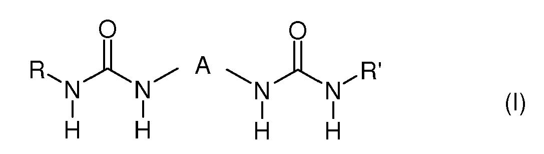 dans laquelle :
- A est un groupement de formule (II) : 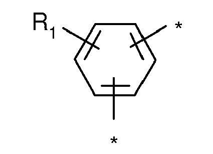 avec R1 étant un radical alkyle C₁ à C₄ linéaire ou ramifié, et les * symbolisant les points de rattachement du groupement A à chacun des deux atomes d'azote du reste du composé de formule générale (I), et
- R et R', identiques ou différents, sont choisis parmi :
   - i) les radicaux de formule (III) : 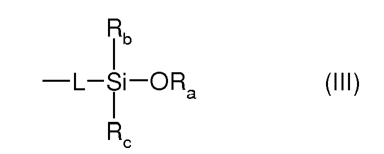
   dans laquelle :
   - L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S;
   - Ra est :
      a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
      b) un radical siliconé de formule : 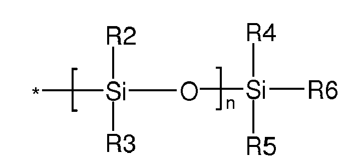
      avec n étant compris entre 0 et 100, notamment entre 1 et 80, voire 2 à 20;
      et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;
- Rb et Rc sont, indépendamment l'un de l'autre, choisis parmi:
   a) les radicaux carbonés, notamment hydrocarbonés (alkyles), linéaires, ramifiés et/ou cycliques, saturés
      ou insaturés, comprenant 1 à 18 atomes de carbone, et pouvant comprendre 1 à 4 hétéroatomes choisis parmi N, 0, Si et S;
   b) les radicaux de formule : 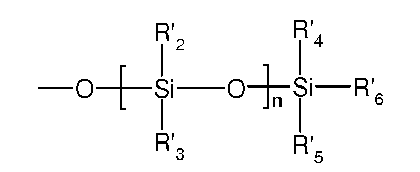 avec n étant compris entre 0 et 100, notamment entre 1 et 80, voire 2 à 20;
      et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
      et
- ii) les radicaux alkyle en C₁ à C₃₀, linéaire, ramifié et/ou cyclique, saturé ou insaturé, et comprenant éventuellement 1 à 3 hétéroatomes choisis parmi O, S, F et N;
   étant entendu que l'un au moins des radicaux R et/ou R' est de formule (III).

On a en effet constaté que cette famille de composés bis-urée pouvait permettre la texturation de milieux siliconés, notamment lorsque les deux radicaux R et R' étaient siliconés, c'est-à-dire de formule (III).
Lorsque l'un des radicaux R ou R' est non siliconé, c'est-à-dire est un radical alkyle tel que défini plus haut, il est possible de texturer avec ces composés les milieux siliconés, mais aussi les milieux carbonés et les milieux comprenant un mélange d'huiles siliconées et carbonées.

Notamment le groupement A peut être de formule : 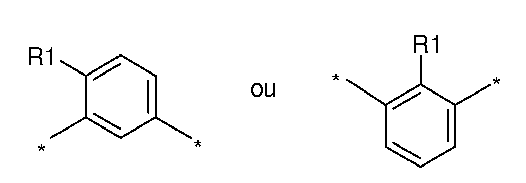 avec R1 et les * étant tels que définis précédemment.

En particulier, R1 peut être un groupement méthyle, ce qui conduit à un groupement A de formule : 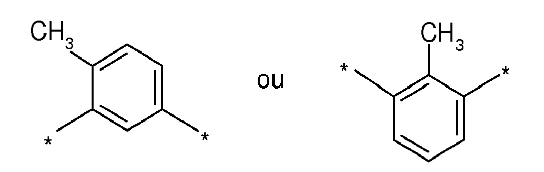 dans laquelle les * sont tels que définis précédemment.
En particulier, les composés selon l'invention peuvent être sous forme de mélange lié au fait que A peut être un mélange de 2,4-tolylène et 2,6-tolylène, notamment dans des proportions (isomère 2,4)/(isomère 2,6) variant de 95/5 à 80/20.

Selon l'invention, l'un au moins des radicaux R et/ou R' doit être de formule (III):

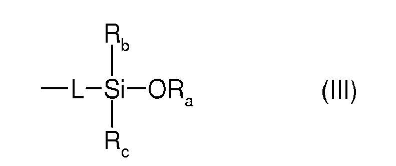

Dans cette formule, L est de préférence un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S. Dans le radical L, la chaîne carbonée peut être interrompue par le/les hétéroatomes et/ou peut comprendre un substituant comprenant ledit/lesdits hétéroatomes.

En particulier, L peut être de structure -(CH₂)n- avec n= 1 à 18, notamment 2 à 12, voire 3 à 8. De préférence, L est choisi parmi les radicaux méthylène, éthylène, propylène, butylène et notamment n-butylène ou octylène.

Le radical L peut également être ramifié, par exemple du type -CH₂-CH(CH₃)-, ce qui conduit au radical de formule (lll) suivant :

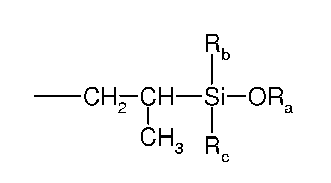

Le radical Ra peut être un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S. La chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou -OSi)-.

Ainsi, le radical Ra peut être de structure -(CH₂)n'-CH₃ avec n'= 0 à 17, notamment 1 à 12, voire de 1 à 6. En particulier, Ra peut être méthyle, éthyle, propyle ou butyle.
Il peut également être de structure -(CH₂)x-O-(CH₂)z-CH₃ ou bien -(CH₂)x-O-(CH₂)y-O-(CH₂)z-CH₃, avec x = 1 à 10, de préférence 2; y=1 à 10 de préférence 2, et z= 0 à 10, de préférence 0 ou 1.
Le radical Ra peut encore être de structure -SiR₄R₅R₆ (cas où n=0), dans laquelle R4, R5 et R6 sont, indépendamment les uns des autres, de préférence, des radicaux alkyle, ayant 1 à 12 atomes de carbone, notamment 1 à 6 atomes de carbone; en particulier R4, R5 et/ou R6 peuvent être choisis parmi méthyle, éthyle, propyle, butyle.
Le radical Ra peut également être un radical siliconé de formule : 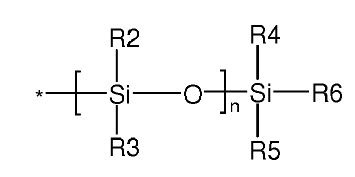 dans laquelle R2 à R6 sont, indépendamment les uns des autres, de préférence, des radicaux alkyle, ayant 1 à 12 atomes de carbone, notamment 1 à 6 atomes de carbone; en particulier R2 à R6 peuvent être choisis parmi méthyle, éthyle, propyle, butyle;
et en particulier un radical : 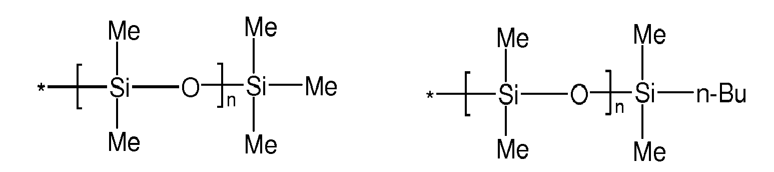 avec n=1 à 100; et encore plus particulièrement un radical :

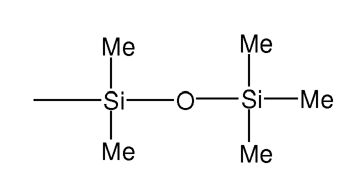

Les radicaux Rb et Rc, identiques ou différents, peuvent être des radicaux carbonés, notamment hydrocarbonés (alkyles), linéaires, ramifiés et/ou cycliques, saturés ou insaturés, comprenant 1 à 18 atomes de carbone, et pouvant comprendre 1 à 8 hétéroatomes choisis parmi N, O, Si et S. Dans ces radicaux, la chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou -OSi)-.
Ainsi, ils peuvent être de structure -(CH₂)m-CH₃ avec m= 0 à 17, notamment 1 à 12, voire 2 à 5; en particulier, Rb et/ou Rc peuvent être méthyl, éthyle, propyle ou butyle;
Ils peuvent également être de structure -O-(CH₂)m'-CH₃ avec m'= 0 à 5, notamment 1 à 4, et en particulier méthoxy ou éthoxy.
Ils peuvent aussi être de structure -O-(CH₂)x-O-(CH₂)z-CH₃ ou -O-(CH₂)x-O-(CH₂)y-O-(CH₂)z-CH₃, avec x = 1 à 10, de préférence 2; y=1 à 10 de préférence 2, et z= 0 à 10, de préférence 0 ou 1.
Ils peuvent encore être de structure : 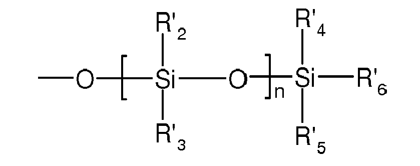 avec n étant compris entre 0 et 100, notamment entre 1 et 80, voire 2 à 20;
et R'2 à R'6 étant, indépendamment les uns des autres, de préférence, des radicaux alkyle, ayant 1 à 12 atomes de carbone, notamment 1 à 6 atomes de carbone; en particulier R'2 à R'6 peuvent être choisis parmi méthyle, éthyle, propyle, butyle.

Lorsqu'ils sont de formule (III), les radicaux R et/ou R' sont de préférence choisis parmi les radicaux suivants : 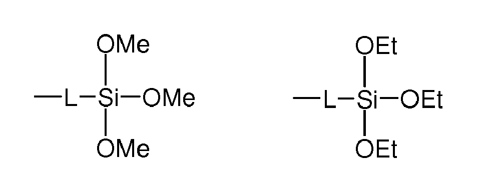 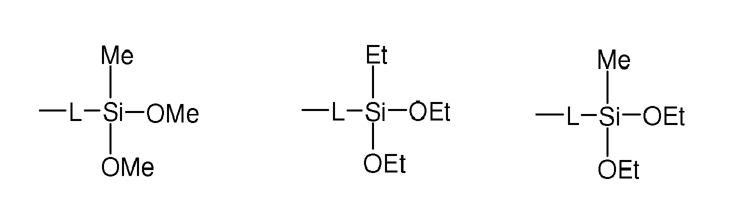 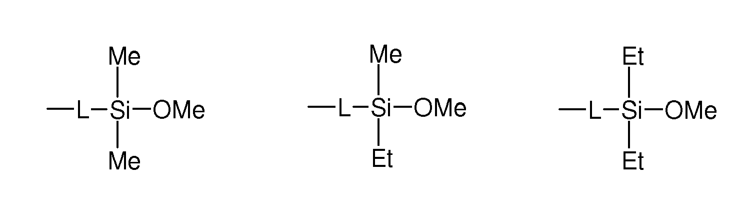 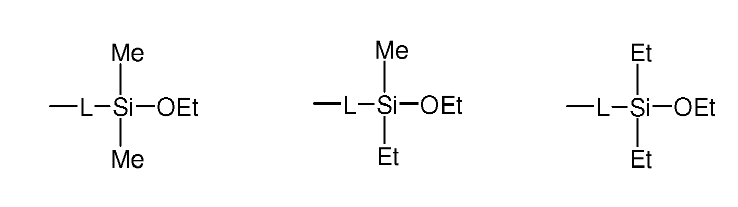 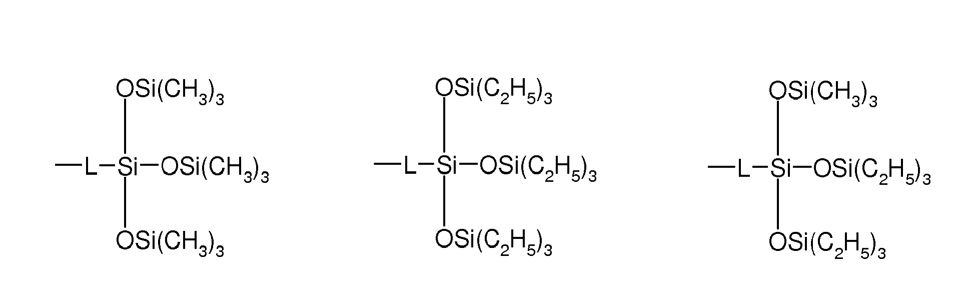 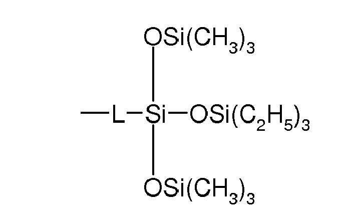 et également ceux de formule : 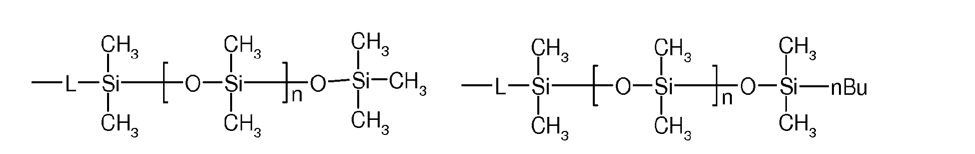 avec n variant de 0 à 100 et en particulier 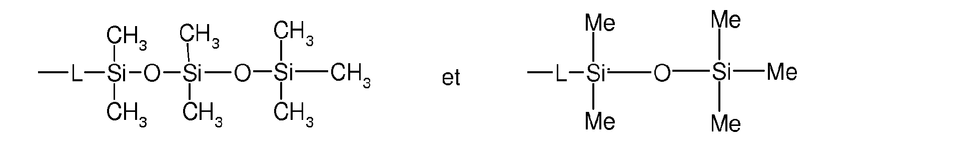 et 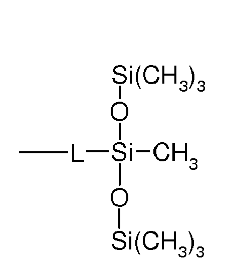 ou
encore

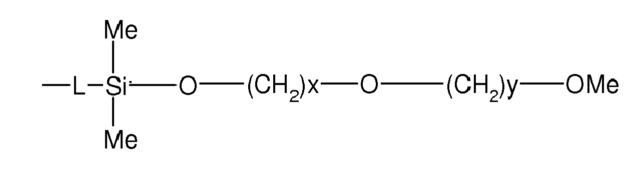

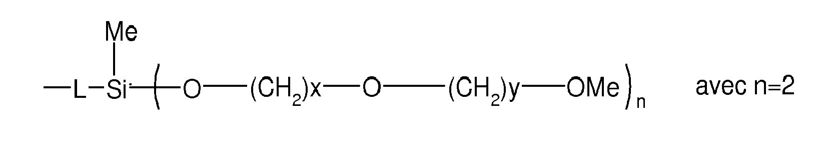

-L-Si(̵O-(CH₂)x-O-(CH₂)y-OMe)ₘ avec m=3

dans lesquelles x = 1 à 10, de préférence 2; et y = 1 à 10, de préférence 2;
et L étant tel que défini ci-dessus.
De préférence, dans ces formules, L est un radical alkylène en C1-C8, linéaire ou ramifié, notamment méthylène, éthylène, propylène, butylène et notamment n-butylène, octylène ou de formule -CH₂-CH(CH₃)-.

Dans un mode de réalisation particulier, R et R', identiques ou différents, sont tous les deux de formule (III).

Dans un autre mode de réalisation, l'un des radicaux R ou R' représente un radical alkyle en C₁ à C₃₀, linéaire, ramifié et/ou cyclique, saturé ou insaturé, et comprenant éventuellement 1 à 3 hétéroatomes choisis parmi O, S, F et N.
Ceci s'avère particulièrement avantageux pour conférer un caractère universel aux composés de formule (I), c'est-à-dire leur permettre de texturer à la fois des milieux carbonés polaires ou apolaires, des milieux siliconés linéaires ou cycliques, des huiles mixtes c'est-à-dire carbonées partiellement siliconées, ainsi que leurs mélanges,.
La chaîne carbonée peut être interrompue par le/les hétéroatomes et/ou peut comprendre un substituant comprenant ledit/lesdits hétéroatomes, notamment sous forme de groupement carbonyle (-CO-), d'un ou plusieurs radicaux hydroxy (-OH), et/ou d'un radical ester -COOR" avec R" = radical alkyle, linéaire ou ramifié, ayant 1 à 8 atomes de carbone.

Ainsi, ledit radical R ou R' peut être un groupement choisi parmi : 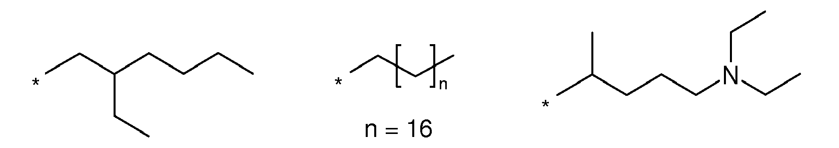 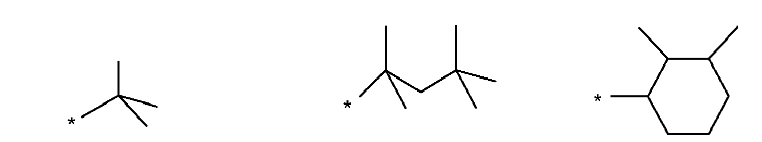 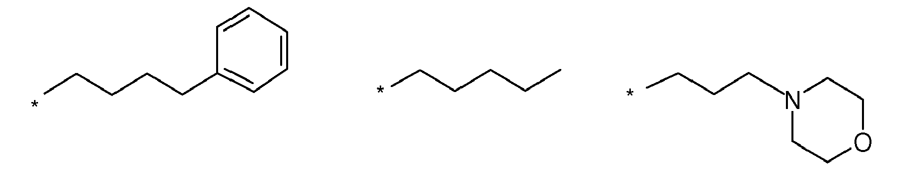 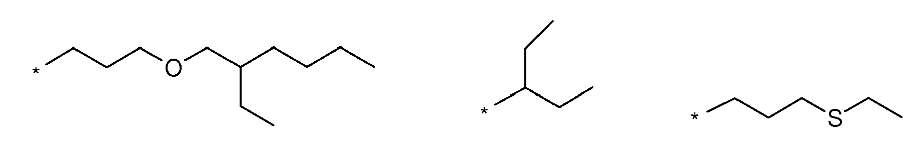 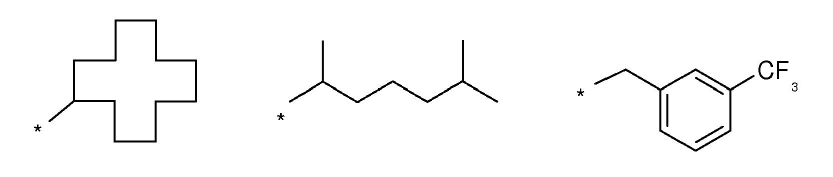 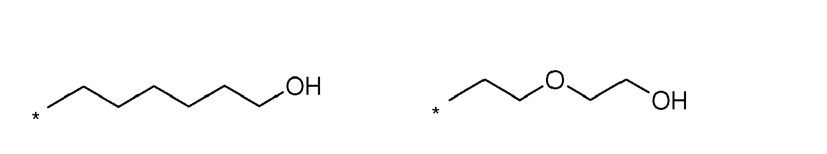 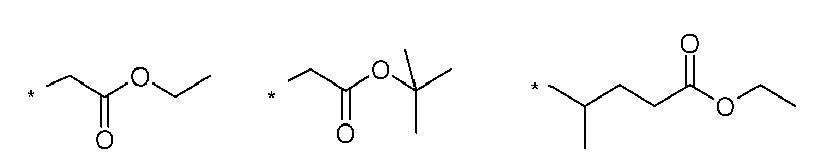 avec * ayant la définition donnée ci-dessus.

Dans un mode de réalisation préféré, R ou R' représente un radical alkyle ramifié, notamment monoramifié, de préférence non cyclique, saturé ou insaturé, comprenant 3 à 16 atomes de carbone, notamment 4 à 12, voire 4 à 8 atomes de carbone, et comprenant éventuellement 1 à 3 hétéroatomes choisis parmi O, S, F et/ou N, de préférence 0 et/ou N.
En particulier, R ou R' peuvent être des radicaux tertio-butyle ou 2-éthylhexyle ou de formule :

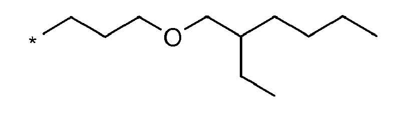

Lorsque le composé de formule (I) comprend un radical R qui est un radical alkyle, et donc un radical R' qui est de formule (III), le rapport entre n_{R} et n_{R'} est de préférence compris entre 5/95 et 95/5, par exemple entre 10/90 et 90/10, en particulier entre 40/60 et 85/15, notamment entre 50/50 et 80/20, voire entre 60/40 et 75/25;
avec n_{R} étant le nombre de moles d'amine NH₂-R et n_{R'} étant le nombre de moles d'amine NH₂-R' utilisées pour préparer le composé de formule (I).

Les composés selon l'invention peuvent se présenter sous forme de sels et/ou d'isomères de composés de formule (I).

Préférentiellement, les composés de formule (I) selon l'invention peuvent être choisis parmi, seuls ou en mélange, les composés suivants, ainsi que leurs sels et isomères :

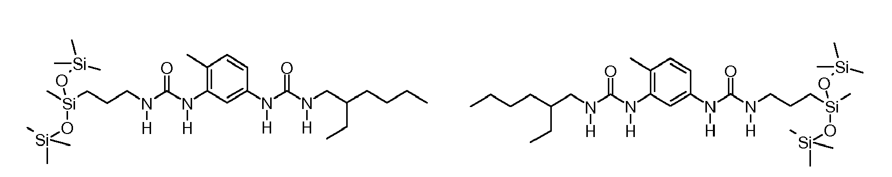

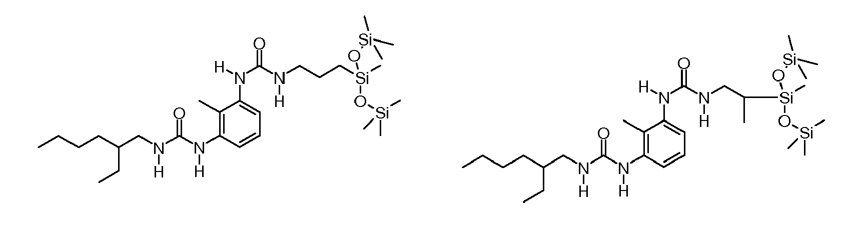

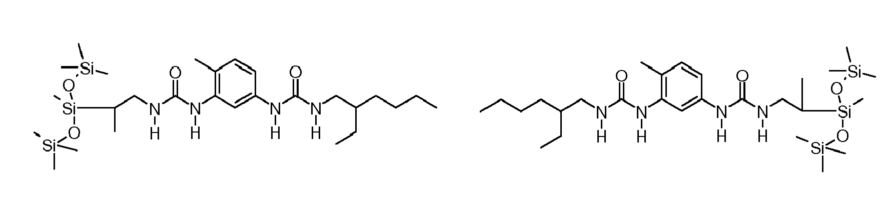

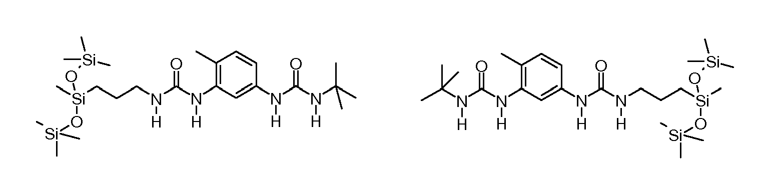

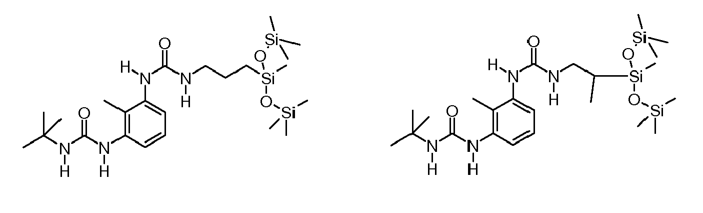

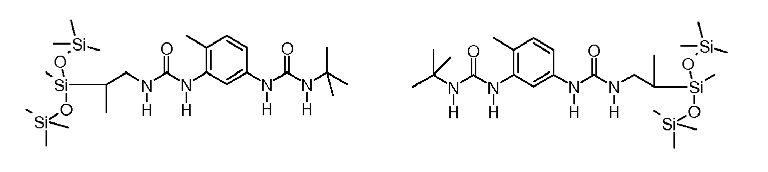

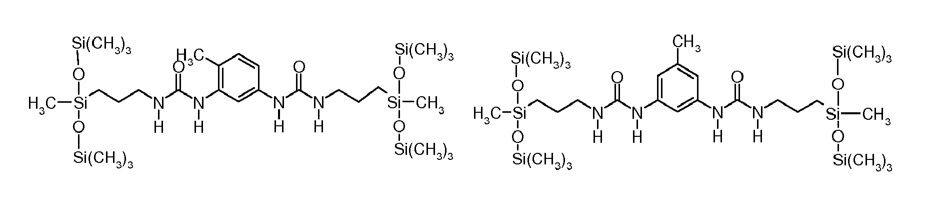

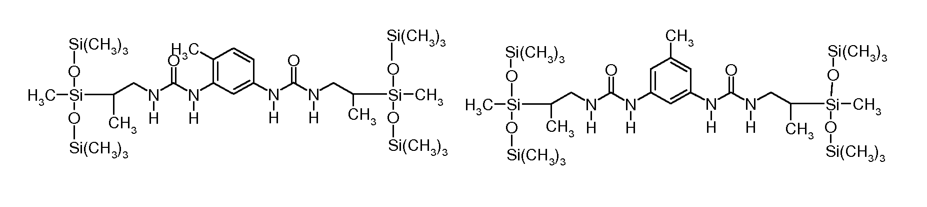

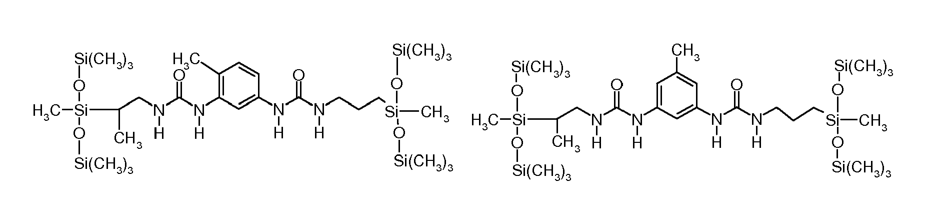

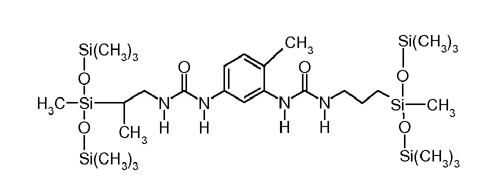

D'une manière générale, les composés de formule générale (I) selon l'invention peuvent être préparés par réaction entre au moins un di-isocyanate de formule OCN-A-NCO, et au moins une amine primaire R-NH₂, avec A et R tels que définis précédemment.
Lorsque les radicaux R et R' sont différents, on fait réagir le diisocyanate avec un mélange d'au moins deux amines primaires : R-NH₂ + R'-NH₂.

Le diisocyanate OCN-A-NCO peut se présenter sous forme de mélange d'isomères de position du substituant R₁ sur le groupement A, notamment dans des proportions 95/5 ou 80/20 (isomère 2,4 TDI)/(isomère 2,6 TDI).

De préférence la/les amines utilisées sont dans un rapport molaire de 2 à 3 équivalents, notamment 2,1 à 2,5, voire 2,2 équivalents pour un équivalent de diisocyanate(s). Le schéma réactionnel général est le suivant :

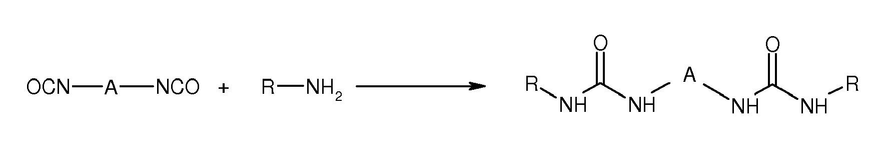

Il est bien évidemment possible d'employer un mélange d'amines primaires, et notamment deux amines primaires.
Dans le cas particulier où deux amines primaires sont utilisées pour la réaction, le rapport molaire n_{R}/n_{R'} peut être compris entre 5/95 et 95/5; ce rapport dépend bien évidemment de la nature chimique de chacune des amines, et sera aisément déterminé par l'homme du métier sur la base de ses connaissances.
Ceci est particulièrement vrai lorsque R et R' sont tous les deux de formule (III).
Lorsque R est un radical alkyle, et donc R' de formule (III), ledit rapport molaire n_{R}/n'_{R'} peut être compris entre 5/95 et 95/5, par exemple entre 15/85 et 90/10, en particulier entre 40/60 et 85/15, notamment entre 50/50 et 80/20;
avec n_{R} étant le nombre de moles d'amine NH₂-R et n_{R'} étant le nombre de moles d'amine NH₂-R' utilisées pour préparer le composé de formule (I).
On peut notamment citer le mélange d'amines primaires commercialisé par Clariant sous la dénomination 'aminopropylbis(triméthylsiloxy)silane', qui correspond à un mélange comprenant 80 à 99,5% en poids, notamment 90 à 99% en poids d'une première amine primaire de formule NH₂-(CH₂)₃-Si[OSi(CH₃₎₃]₂Me et 0,5 à 20% en poids, notamment 1 à 10% en poids d'une deuxième amine primaire, de formule NH₂-CH₂-CH(CH₃)-Si[OSi(CH₃)₃]₂Me.

La réaction est généralement effectuée sous atmosphère inerte par exemple sous argon, en milieu anhydre avec par exemple une température du milieu réactionnel qui est maintenue entre 15°C et 40°C.
Le diisocyanate peut être mis en solution dans un solvant anhydre tel que la tétrahydrofurane, la 2-méthyl-tétrahydrofurane, la N-méthylpyrrolidone, l'acétate de butyle ou encore la méthyl-éthylcétone à une concentration pouvant aller de 1 à 30% en poids, de préférence de 2 à 20%, voire de 4 à 10% en poids.
Une solution contenant la/les amines peut être préparée dans le même solvant que le diisocyanate, à une concentration pouvant aller de 0,1 à 99,9% en poids.
La température du milieu réactionnel ne doit préférentiellement pas dépasser 40°C et la concentration de l'amine ainsi que la vitesse d'addition de la solution contenant l'amine sont préférentiellement ajustées à cette nécessité.
Le milieu réactionnel peut être laissé sous agitation par exemple pendant 30 minutes à 12 heures. Le contrôle de l'avancement de la réaction peut être réalisé par spectrométrie infra-rouge (notamment en observant la disparition de la bande NCO entre 2250 et 2280 cm⁻¹).
En fin de réaction, on peut verser le milieu réactionnel dans une grande quantité d'eau acide (notamment un pH 3-4 par HCl). On obtient alors un précipité, généralement blanc, qui est filtré, lavé par exemple plusieurs fois notamment à l'eau, et séché sous pression réduite, notamment sous vide ou lyophilisé.

Le précipité correspond au composé de formule (I) attendu, ou au mélange de composés de formule (1) attendu, et peut être caractérisé par spectrométrie RMN (¹H et ou ¹³C) et/ou par HPLC couplé à la masse.

Le composé peut être utilisé tel quel pour la texturation du milieu huileux considéré.
En effet, les composés de formule (1), seuls ou en mélange en toutes proportions, peuvent se solubiliser dans une grande diversité d'huiles et s'avèrent donc efficaces pour texturer les huiles ou mélange d'huiles considérées, en lui conférant les propriétés physiques et/ou chimiques souhaitées.
Le composé de formule (I) est avantageusement soluble à une température inférieure ou égale à 50°C, voire inférieure ou égale à 30°C, et notamment à température ambiante (25°C), dans les phases grasses liquides usuellement employées en cosmétique, et donc dans la phase grasse à texturer.

Le composé selon l'invention trouve donc une application toute particulière dans les compositions cosmétiques ou pharmaceutiques usuelles, notamment en tant qu'agent texturant, épaississant, voire gélifiant, de la phase grasse liquide comprise dans ladite composition.

Ledit composé de formule (1), seul ou en mélange, peut être présent dans lesdites compositions en une quantité efficace, c'est-à-dire en une quantité nécessaire et suffisante pour obtenir la texturation de la phase grasse liquide considérée dans la composition selon l'invention.

Par "phase grasse liquide texturée", on entend que ladite phase grasse prend l'état d'un gel ou d'un liquide épaissi. Elle peut s'écouler sous son propre poids. Elle peut se déformer à volume constant si on exerce une contrainte.
Cette texturation se traduit en particulier par une augmentation de la viscosité due notamment à l'introduction d'au moins un composé de formule (I).

Ainsi, les compositions selon l'invention peuvent comprendre de 0,01 à 20% en poids, notamment de 0,05 à 15% en poids, voire de 0,1 à 10% en poids, mieux de 1 à 8% en poids, et encore mieux de 2 à 5% en poids, de composé(s) de formule (I) par rapport au poids total de la composition.
La quantité efficace de composé(s) de formule (I) peut notamment représenter de 0,01 à 20% en poids, notamment de 0,05 à 15% en poids, voire de 0,1 à 10% en poids, mieux de 1 à 8% en poids, et encore mieux de 2 à 5% en poids, par rapport au poids de ladite phase grasse liquide.
Il est clair que cette quantité efficace est susceptible de varier significativement selon, entre autre, la nature du composé dérivé bis-urée, le fait qu'il soit utilisé à l'état pur ou en mélange avec d'autres dérivés bis-urée de formule (I), et la nature de la phase grasse liquide.

### Bis-urées hydrocarbonées

Selon un mode particulier de réalisation, les composés de type bis-urée siliconés décrits précédemment peuvent être en mélange avec d'autres composés bis-urées non siliconés. Les composés de bis-urée non siliconés peuvent, selon un premier aspect, répondre à la formule générale (II) suivante : 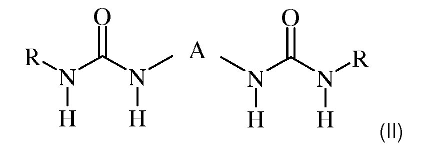 dans laquelle :
- A est un groupement de formule : 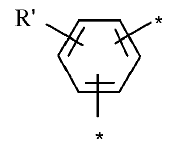 avec R' étant un radical alkyle C₁ à C₄ linéaire ou ramifié et les * symbolisant les points de rattachement du groupement A à chacun des deux atomes d'azote du reste du composé de formule générale (II), et
- R est un radical alkyle en C₆ à C₁₅, mono-ramifié, non cyclique, saturé ou insaturé et dont la chaîne hydrocarbonée est éventuellement interrompue par 1 à 3 hétéroatomes choisis parmi O, S et N, ou l'un de ses sels ou isomères.

Selon un mode de réalisation préféré de l'invention, le groupement figuré par A est un groupement de formule : 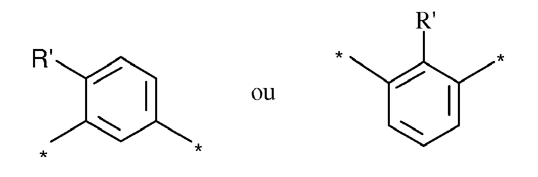 avec R' et les * étant tels que définis précédemment.

En particulier, R' peut être un groupement méthyle, et le groupement A est alors plus particulièrement un groupement de formule : 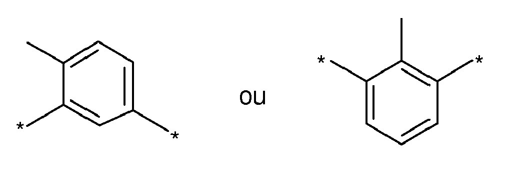 avec les * étant tel que défini précédemment.

Selon un premier mode de réalisation de l'invention, R peut être choisi parmi les radicaux mono-ramifiés de formule générale CₙH₂ₙ₊₁, n étant un entier variant de 6 à 15, en particulier de 7 à 9 voire égal à 8.
Ainsi, les deux groupements R du composé de formule (II) peuvent figurer respectivement un groupement : 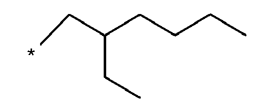 avec * symbolisant le point de rattachement de chacun des groupements R à chacun des atomes d'azote du reste du composé de formule générale (II).

Selon un deuxième mode de réalisation de l'invention, R peut être choisi parmi les radicaux mono-ramifiés de formule générale Cₘ₋ₚH₂ₘ₊₁₋₂ₚXₚ, p étant égal à 1, 2 ou 3, de préférence égal à 1, m étant un entier variant de 6 à 15, de préférence de 10 à 14, en particulier de 10 à 12, voire égal à 11 et X représentant les atomes de soufre et/ou d'oxygène, en particulier les atomes d'oxygène.
Plus particulièrement, R peut être un radical de formule Cₘ,H₂ₘ,X-(Cₚ,H₂ₚ,X')ᵣ-CₓH₂ₓ₊₁, dans laquelle X et X' sont indépendamment l'un de l'autre un atome d'oxygène ou de soufre, de préférence d'oxygène, r vaut 0
ou 1, m', p' et x sont des entiers tels que leur somme varie de 6 à 15, en particulier de 10 à 12, voire est égale à 11 et étant entendu qu'au moins une des chaînes carbonées C_{m'}H_{2m'}, C_{p'}H_{2p'}, ou CₓH₂ₓ₊₁ est ramifiée.
De préférence c'est la chaîne CₓH₂ₓ₊₁ qui est ramifiée, de préférence r égal 0, de préférence m' est un entier variant de 1 à 10, notamment de 2 à 6, en particulier égal à 3, et/ou de préférence x est un entier variant de 4 à 16, notamment de 6 à 12, en particulier égal à 8.
Ainsi, les deux groupements R du composé de formule (I) peuvent figurer respectivement un groupement : 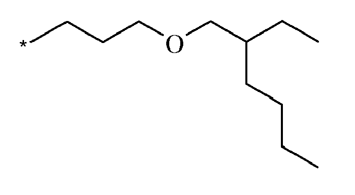 avec * symbolisant le point de rattachement de chacun des groupements R à chacun des atomes d'azote du reste du composé de formule générale (I).
De tels composés peuvent être présents dans les compositions selon l'invention en mélanges avec des isomères, notamment des isomères de position sur le groupement A, notamment dans des proportions 95/5 ou 80/20.

Comme il ressort des exemples ci-après, la présence de l'un ou l'autre de ses radicaux dans la molécule de formule générale (II) s'avère particulièrement avantageuse pour conférer un caractère universel, au sens de l'invention, aux dérivés bis-urée non siliconés correspondants.
A titre représentatif et non limitatif des composés convenant tout particulièrement à l'invention, on peut plus particulièrement citer les composés suivants, utilisés purs ou en mélange : 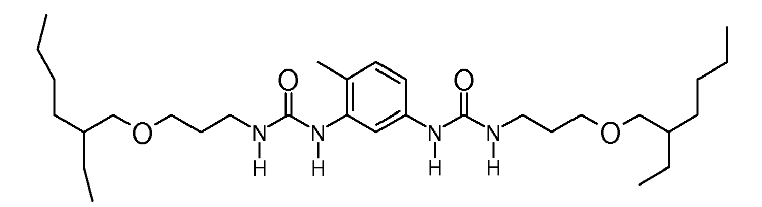 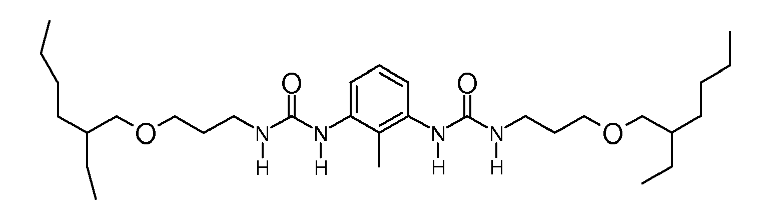 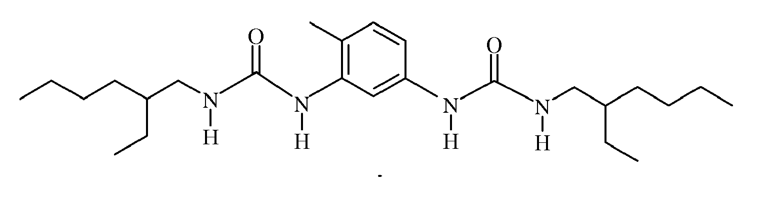 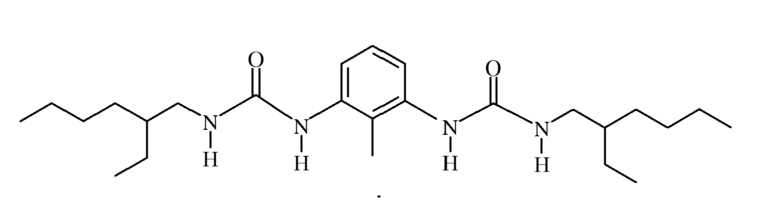 et leurs sels.
Par « conviennent particulièrement dans le cadre de l'invention », on entend que le composé de formule générale (II), seul ou en mélange en toutes proportions, peut se solubiliser dans une grande diversité d'huiles et qu'il s'avère efficace pour texturer ladite huile ou mélange d'huiles considéré(e) et donc lui conférer les propriétés physiques et/ou chimiques souhaitées.

Par « physiologiquement acceptable », on désigne un milieu dénué de toxicité et compatible avec une application sur la peau, les lèvres et/ou les phanères d'êtres vivants et en particulier d'êtres humains. En conséquence, les compositions selon l'invention sont dénuées de composés incompatibles et/ou non tolérés pour une application sur la peau, les lèvres et/ou les phanères.
Au sens de la présente invention, le terme « quantité efficace » désigne la quantité nécessaire et suffisante pour obtenir la texturation de l'huile ou mélange d'huiles considéré dans la composition selon l'invention.
Par « phase grasse liquide texturée », on entend que la phase grasse prend l'état d'un gel ou d'un liquide épaissi. Elle peut s'écouler sous son propre poids. Elle peut se déformer à volume constant si on exerce une contrainte.
Cette texturation se traduit en particulier par une augmentation de la viscosité due notamment à l'introduction d'au moins un composé de formule générale (1).
Par exemple, les compositions selon l'invention peuvent contenir de 0,01 à 20 % en poids, notamment de 0,1 à 15 %, voir de 1 à 10, encore mieux de 2 à 8 %, en poids de composé(s) de formule (II) par rapport au poids total de la composition.
La quantité efficace de composé(s) de composé(s) de formule (II), peut représenter de 0,01 à 20 %, notamment de 0,05 à 10 %, par exemple de 0,1 à 5 %, voire de 0,05 à 3 % en poids de la phase grasse liquide.
Pour des raisons évidentes, cette quantité efficace est susceptible de varier significativement selon d'une part la nature du substituant R du dérivé bis-urée, sa position, le fait qu'il soit utilisé à l'état pur ou en mélange avec d'autres dérivés bis-urée de formule (II) ou non, et d'autre part la nature de la phase grasse liquide.
D'une manière générale, le composé de formule générale (II) selon l'invention dérive de la réaction entre au moins un di-isocyanate de formule : 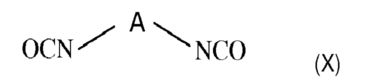 et une amine primaire de formule : 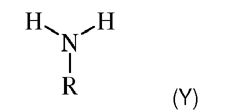 avec A et R tels que définis précédemment.
Le cas échéant, les différents diisocyanates (X) peuvent être des isomères de positions du susbtituant R' sur le groupement A, notamment dans des proportions 95/5 ou 80/20.
De préférence l'amine (Y) utilisée est dans un rapport molaire de 2 à 3 équivalents, notamment 2,1 à 2,5, voire 2,2 équivalents pour un équivalent de diisocyanate(s) (X). Le schéma réactionnel général est le suivant :

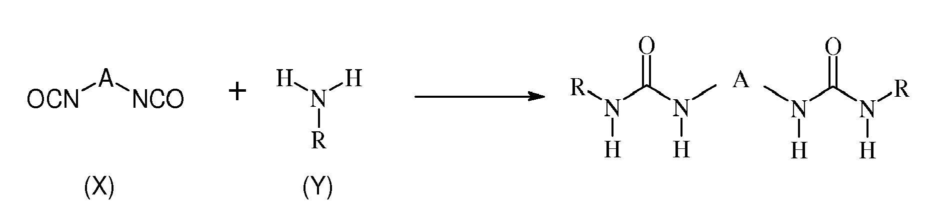

La réaction est généralement effectuée sous atmosphère inerte par exemple sous argon, en milieu anhydre avec par exemple une température du milieu réactionnel qui est maintenue entre 15 °C et 40 °C, de préférence entre 18 °C et 25 °C.
Le(s) diisocyanate(s) (X) peut(peuvent) être mis en solution dans un solvant anhydre tel que la tétrahydrofurane, la 2-méthyl-tétrahydrofurane, la N-méthylpyrrolidone, l'acétate de butyle ou encore la méthyl-éthylcétone à une concentration pouvant aller de 1 à 30 % en poids, de préférence de 2 à 20 %, voire de 4 à 10 % en poids.
Une solution contenant l'amine (Y) est généralement préparée dans le même solvant que le(s) diisocyanate(s) (X) à une concentration allant par exemple de 0,1 à 99,9 % en masse. La température du milieu réactionnel ne doit préférentiellement pas dépasser 40 °C et la concentration de l'amine ainsi que la vitesse d'addition de la solution contenant l'amine (Y) doivent donc être préférentiellement ajustées à cette nécessité. Le milieu réactionnel peut être laissé sous agitation par exemple pendant 30 min à 12 heures. Le contrôle de l'avancement de la réaction peut être réalisé par spectrométrie infra-rouge (notamment en observant la disparition de la bande NCO entre 2250 et 2280 cm⁻¹). Par exemple, en fin de réaction, on verse le milieu réactionnel dans une grande quantité d'eau acide (notamment un pH 3-4 par HCl). On obtient alors un précipité qui est filtré, lavé par exemple plusieurs fois notamment à l'eau et séché sous pression réduite, notamment sous vide ou lyophilisé. Le précipité correspond aux composés de formule (I) attendu(s), et peut être caractérisé par spectrométrie RMN (¹H et ou ¹³C) et/ou par HPLC et peut être utilisé tel quel pour la texturation du milieu huileux considéré.
La bis-urée ou le mélange de bis-urées est avantageusement soluble à une température inférieure ou égale à 50 °C, voire inférieure ou égale à 30 °C, et notamment à température ambiante, dans la phase grasse liquide à texturer.

Selon un autre aspect de l'invention, les dérivés bis-urée non siliconés formule (III) suivante : 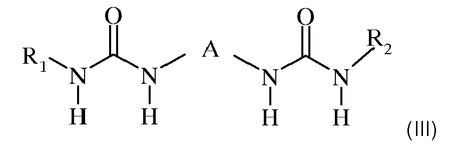 dans laquelle :
- A est un groupement de formule : 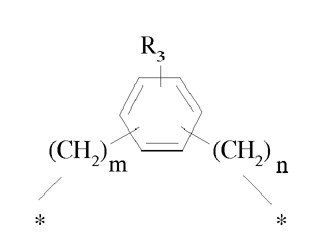 avec
- R₃ étant un atome d'hydrogène ou un radical alkyle en C₁ à C₄ linéaire ou ramifié,
- n et m étant, indépendamment l'un de l'autre, égaux à 0 ou 1, et
- * symbolisant le point de rattachement du groupement A aux deux atomes d'azote du reste du composé de formule générale (III),
- R₁ est un radical carboné en C₃ à C₁₅, ramifié, non cyclique, saturé ou insaturé et contenant éventuellement de 1 à 3 hétéroatomes choisis parmi O, S, F et N et/ou un carbonyle, et leurs combinaisons,
- R₂ est différent de R₁ et est choisi parmi les radicaux alkyles en C₁-C₂₄, saturé ou insaturé, linéaire, ramifié ou cyclique, contenant éventuellement de 1 à 3 hétéroatomes choisis parmi O, S, F et N, et éventuellement substitué par :
   - 1, 2 ou 3 radicaux hydroxy,
   - un radical ester (-COOR₄), avec R₄ étant un radical alkyle, linéaire ou ramifié, ayant de 1 à 8, notamment 1 à 6, voire 2 à 4 atomes de carbone ;
   - un radical cyclique saturé, insaturé ou aromatique ayant de 5 à 12 atomes de carbone, en particulier un radical phényle éventuellement substitué par un ou plusieurs radicaux identiques ou différents choisis parmi les radicaux alkyle en C₁-C₄, trifluorométhyle,
      ou un dérivé morpholine, et/ou
   - un ou plusieurs radicaux alkyles linéaires ou ramifiés en C₁-C₄,
ou l'un de ses sels ou isomères.

En particulier n et m sont égaux, et plus particulièrement égaux à zéro et R₃ est un radical R'₃, tel que défini ci-après. Ainsi, de manière préférée, A représente un groupement

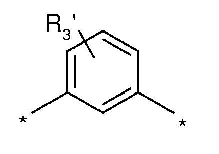

Avec R₃' étant un radical alkyle en C₁ à C₄ linéaire ou ramifié et * symbolisant les point de rattachement du groupement A aux deux atomes d'azote du reste du composé de formule générale (III).

Selon une variante de l'invention, le composé de formule générale (III) comprend à titre de A, au moins un groupement choisi parmi : 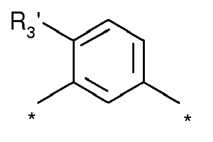 ou 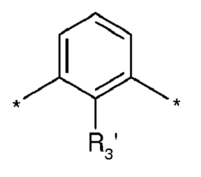 avec R₃' et * étant tels que défini précédemment.
En particulier, R₃' peut être un groupement méthyle, et dans ce cas le groupement A représente un groupement 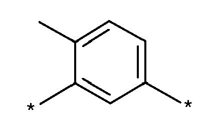 ou 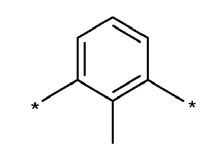
* étant tel que défini précédemment.
En particulier, les composés sont tels que A est un mélange de 2,4-tolylène et 2,6-tolylène notamment dans des proportions (isomère 2,4)/(isomère 2,6) variant de 95/5 à 80/20.
Selon un mode de réalisation de l'invention, le composé de formule générale (lll) comprend à titre de R₁, un radical en C₆-C₁₅ ramifié.

Selon un mode de réalisation de l'invention, le composé de formule générale (III) comprend à titre de R₁, un groupement choisi parmi : 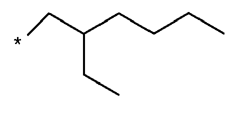 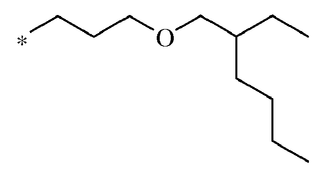 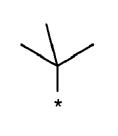 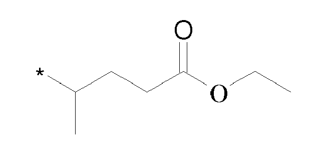 avec * symbolisant le point de rattachement du groupement R₁ à l'azote du reste du composé de formule générale (III).
Comme il ressort des exemples ci-après, la présence de l'un et/ou l'autre de ses deux radicaux dans la molécule de formule générale (III) s'avère particulièrement avantageuse pour conférer un caractère universel au sens de l'invention, aux dérivés bis-urée asymétriques correspondants.
En ce qui concerne R₂ qui est différent de R₁, il peut être avantageusement choisi parmi les groupements suivants : 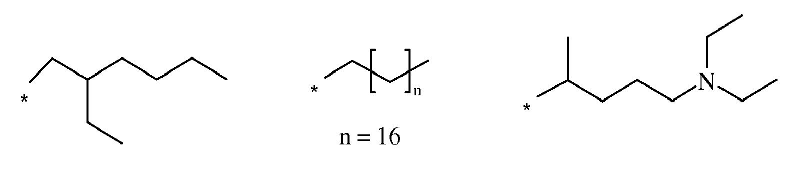 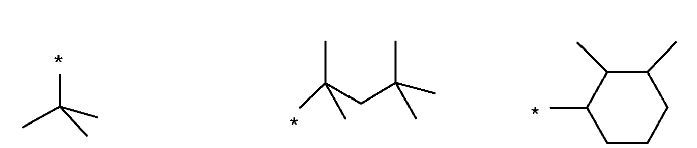 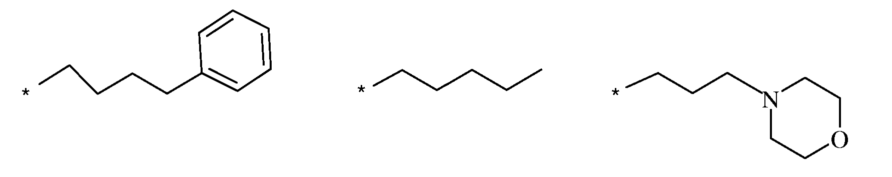 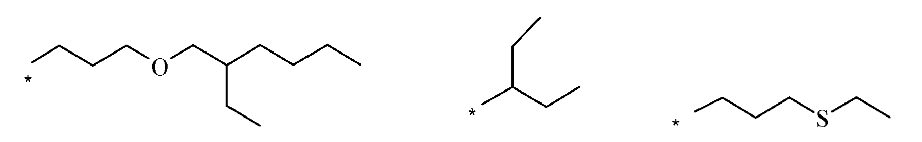 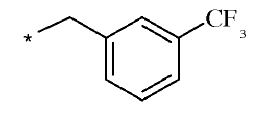 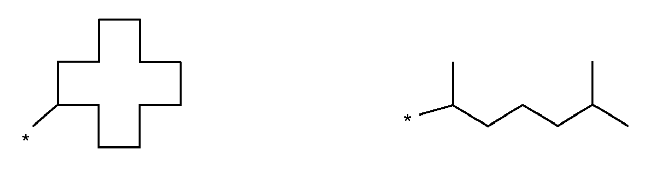 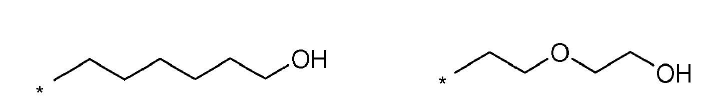 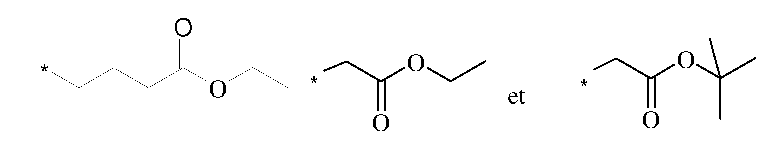 avec * symbolisant le point de rattachement du groupement R₂ à l'azote du reste du composé de formule générale (III).
A titre représentatif et non limitatif des composés convenant tout particulièrement à l'invention, on peut plus particulièrement citer les composés suivants :

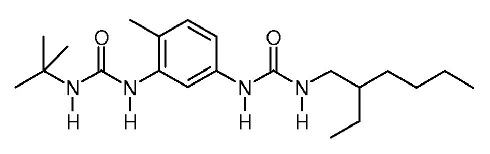

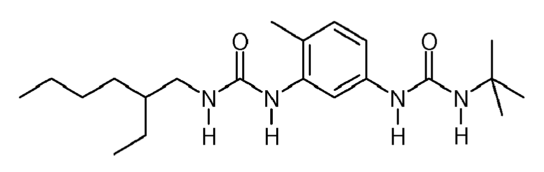

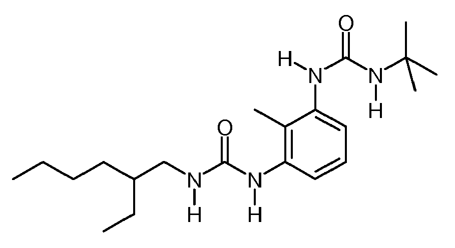

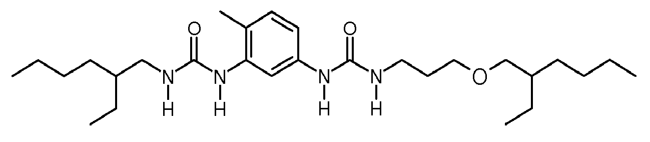

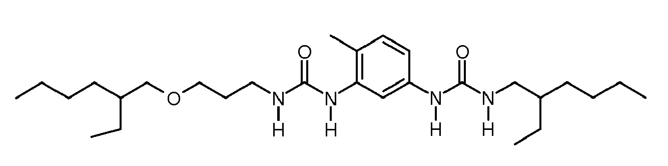

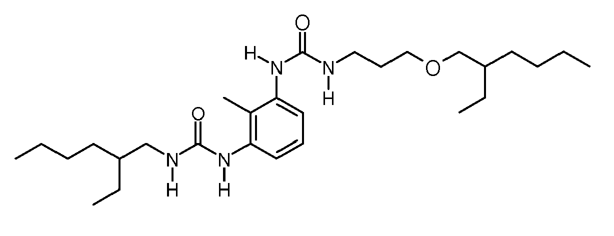

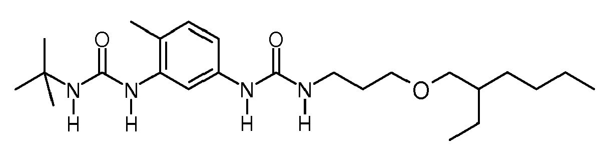

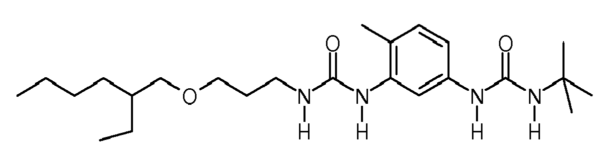

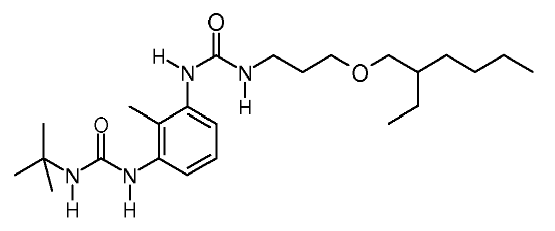

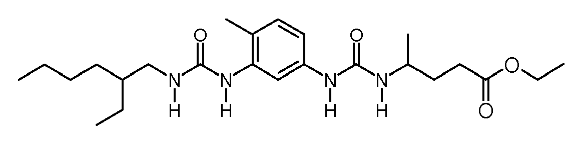

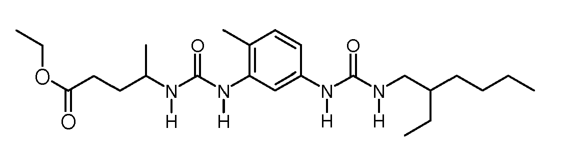

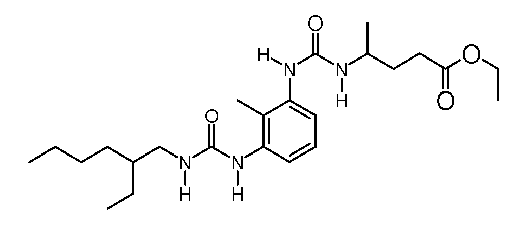

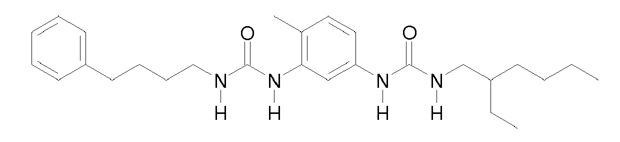

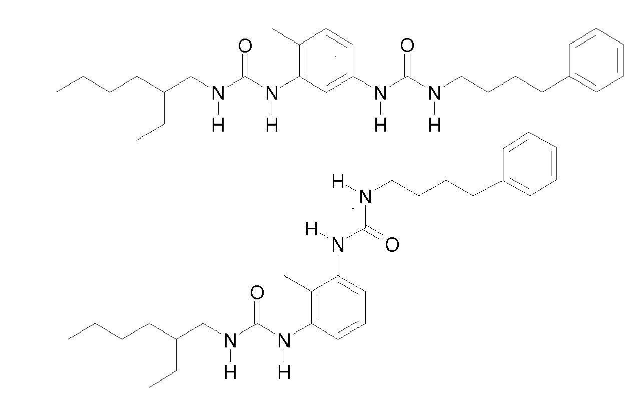

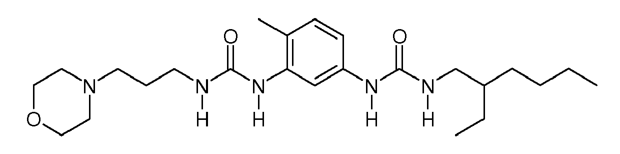

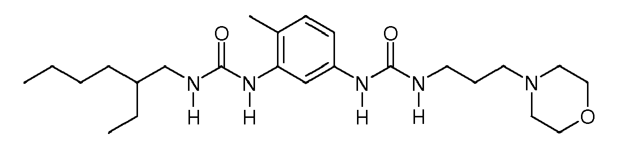

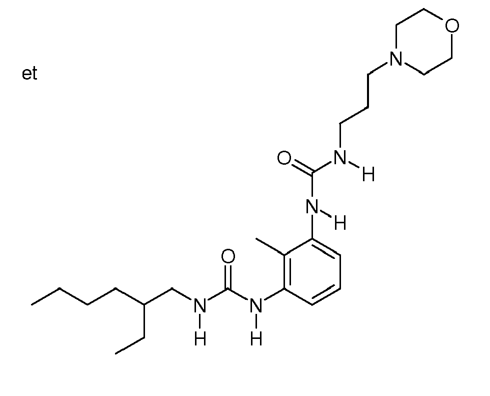

Par « conviennent particulièrement dans le cadre de l'invention », on entend que le composé de formule générale (III), seul ou en mélange en différentes proportions, peut se solubiliser à température ambiante dans plusieurs huiles cosmétiques et qu'il s'avère efficace pour gélifier ladite huile ou mélange d'huiles considéré(e) et donc lui conférer les propriétés physiques et/ou chimiques souhaitées.
Au sens de la présente invention, le terme « quantité efficace » désigne la quantité nécessaire et suffisante pour obtenir la texturation de l'huile ou mélange d'huiles considéré dans la composition selon l'invention.
Cette texturation se traduit en particulier par une augmentation de la viscosité qui peut être due à l'introduction d'au moins un composé de formule générale (III).
Par exemple, les compositions selon l'invention peuvent contenir de 0,0001 à 5 % en poids de bis-urée non siliconés, notamment de 0,001 à 1 %, voir de 0,004 à 0,5 % en poids d'au moins une bis-urée asymétrique par rapport au poids total de la phase grasse liquide
Pour des raisons évidentes, cette quantité efficace est susceptible de varier significativement selon d'une part la nature des substituants R₁ et/ou R₂ du dérivé bis-urée, l'isomère de position, le fait qu'il est utilisé à l'état pur ou en mélange avec d'autres dérivés bis-urée de formule (III) ou non, et d'autre part la nature de la phase huileuse.
Pour des raisons notamment liées à la procédure de préparation, les dérivés bis-urées asymétriques de formule générale (III), peuvent être mis en oeuvre dans le cadre de la présente invention sous la forme d'un mélange de dérivés de formule (III) entre eux, et/ou avec les deux formes de dérivés bis-urées symétriques correspondantes. On entend au sens de la présente invention par bis-urées symétriques, les bis-urées selon la formule (III) avec des radicaux R₁ et R₂ identiques.
D'une manière générale, le composé de formule générale (III) selon l'invention dérive de la réaction entre au moins un diisocyanate de formule : 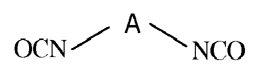 et au moins deux amines primaires distinctes de formule : 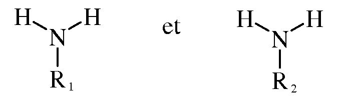 avec A, R₁ et R₂ tels que définis précédemment.

Le cas échéant, les différents diisocyanates peuvent être des isomères de positions du susbtituant R₃ sur le groupement A, notamment dans des proportions 95/5 ou 80/20.
Par au moins deux amines primaires distinctes, on entend qu'on peut y adjoindre d'autres amines primaires Rₓ-NH₂ dans lesquelles Rₓ répond aux définitions proposées pour R₁ et R₂.

Le nombre d'amines utilisées pour la réaction peut être supérieur ou égal à 2, par exemple peut varier de 2 à 20, et plus particulièrement entre 3 et 10. Pour faciliter la préparation de la composition et la caractérisation du mélange ainsi obtenu, il est avantageux de se limiter à l'utilisation de deux amines.
De préférence l'ensemble des amines utilisées est dans un rapport molaire de 2 à 3 équivalents, notamment 2,1 à 2,5, voire 2,2 équivalents pour un équivalent de diisocyanate(s).
Dans le cas particulier où seulement deux amines primaires sont utilisées pour la réaction, le rapport molaire n(R₁)/n(R₂) peut être compris entre 1/99 et 99/1, plus particulièrement entre 5/95 et 95/5, voire entre 10/90 et 90/10 avec n(R₁) correspondant au nombre de moles de : 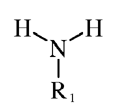 et n(R₂) correspondant au nombre de moles de : 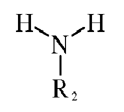 et avec R₁, R₂, tels que définis précédemment.

La réaction est généralement effectuée sous atmosphère inerte par exemple sous argon, en milieu anhydre avec par exemple une température du milieu réactionnel qui est maintenue inférieure à 50 °C, voire comprise entre 15 °C et 40 °C, de préférence entre 18 °C et 25 °C.
Le(s) diisocyanate(s) peut(peuvent) être mis en solution dans un solvant anhydre tel que la tétrahydrofurane, la 2-méthyl-tétrahydrofurane, la N-méthylpyrrolidone, l'acétate de butyle ou encore la méthyl-éthylcétone à une concentration pouvant aller de 1 à 30 % en poids, de préférence de 2 à 20 %, voire de 4 à 10 % en poids.
Une solution contenant les amines est généralement préparée dans le même solvant que le(s) diisocyanate(s) à une concentration allant par exemple de 0,1 à 99,9 % en masse. La température du milieu réactionnel ne doit préférentiellement pas dépasser 40 °C et la concentration des amines ainsi que la vitesse d'addition de la solution contenant les amines doivent donc être préférentiellement ajustées à cette nécessité. Le milieu réactionnel peut être laissé sous agitation par exemple pendant 30 min à 12 heures. Le contrôle de l'avancement de la réaction peut être réalisé par spectrométrie infra-rouge (notamment en observant la disparition de la bande NCO entre 2250 et 2280 cm⁻¹). Par exemple, en fin de réaction, on verse le milieu réactionnel dans une grande quantité d'eau acide (notamment un pH 3-4 par HCl). On obtient alors un précipité qui est filtré, lavé par exemple plusieurs fois notamment à l'eau et séché sous pression réduite, notamment sous vide ou lyophilisé.
En conséquence, à l'issue de cette réaction, on obtient conjointement avec le dérivé asymétrique de formule (III) attendu, au moins l'une des deux formes suivantes : 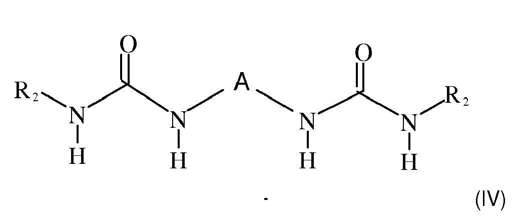 et 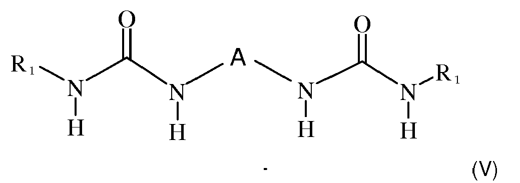 avec A, R₁ et R₂ tels que définis précédemment, avec A étant identique dans les formules (III), (IV), (V), R₁ étant identique en formules (III) et (V) et R₂ étant identique en formules (III) et (IV).
Le précipité correspond aux composés de formule (III) attendu(s), et peut être caractérisé par spectrométrie RMN (¹H et ou ¹³C) et/ou par HPLC et peut être utilisé tel quel pour la texturation du milieu huileux considéré.
En fin de réaction, le mélange de bis-urées (III), (IV) et (V) est isolé et peut être utilisé tel quel pour la gélification du milieux huileux souhaité.
Auquel cas, le composé de formule (III) est mis en oeuvre dans la phase grasse liquide sous la forme d'un mélange avec les composés de formule générale (IV) et (V).
Le mélange de bis-urées est avantageusement soluble à une température inférieure ou égale à 50 °C, voire inférieure ou égale à 30 °C, et notamment à température ambiante, dans la phase grasse liquide à texturer.

### Huiles compatibles bis urée

La composition selon l'invention comprend au moins une phase grasse liquide comprenant au moins une huile ayant un paramètre de solubilité δₐ compris entre 0 et 7,00 (J/cm³)^{½}, et de préférence entre 0 et 5,00 (J/cm³)^{½}.

Le paramètre de solubilité δₐ est calculé par la relation δₐ = (δₚ² + *δ*ₕ²)^{½} dans laquelle les paramètres δₚ, δₕ correspondent aux paramètres de solubilité de HANSEN :
- δ_{P} caractérise les forces d'interactions de DEBYE entre dipôles permanents ;
- δₕ caractérise les forces d'interactions spécifiques (type liaisons hydrogène, acide/base, donneur/accepteur, etc.) ;

Les paramètres δ_{P} et δₕ sont généralement exprimés en (J/cm³)^{½}. Ils sont déterminés à température ambiante (25°C) et en particulier selon la méthode de calcul indiquée dans le document brevet JP-A-08-109121.

La définition des paramètres de solubilité selon HANSEN est bien connue de l'homme du métier, et notamment décrite dans l'article de C. M. HANSEN : "The three dimensional solubility parameters" J. Paint Technol. 39, 105 (1967). Ces paramètres sont aussi décrits dans le document JP-A-08-109121 de KAO et le document de D.W. Van KREVELEN "Properties of polymers" (1990), p. 190.

L'huile ayant un paramètre de solubilité δₐ compris entre 0 et 7,00 (J/cm³)^{1/2} peut être choisie parmi les huiles hydrocarbonées ou les huiles siliconées.

Les huiles ayant un paramètre de solubilité δₐ compris entre 0 et 7,00 (J/cm³)^{1/2} peuvent notamment être choisies parmi le squalane, les polyisobutylène de poids moléculaire entre 250 et 800 g/mol tels que l'huile de parléam, l'huile de jojoba, le propionate d'arachidyle, le stearoyl-stearate d'octyl dodecyl, l'huile d'arara, le copolymère de PVP / Hexadécène, l'érucate d'oléyle, le stéarate d'octyl dodecyl, le palmitate d'isostéaryle, le stéarate d'isocétyle, le di-décène, le polydécène hydrogéné, l'isostéarate d'isostéaryle, la dicaprylyl éther, l'isoéicosane, le dioctyldodécyl dimer dilinoléate, le myristate d'octyldodécyle, le trilinoléate de triisostéaryle, le néodécanoate d'octyldodécyle, l'octyldodécyl octanoate, l'isononanoate d'isostéaryle, l'isostérate d'isofol 24, l'érucate d'oxtyldodécyle, l'huile de sésame, le myristate d'isopropyle, les huiles de silicone linéaires telles que les polydiméthylsiloxanes, le stéarate d'isobutyle, le palmitate d'isopropyle, le stéaryl heptanoate, le stéarate d'isopropyle, les triglycérides caprylique/capric, le propylène glycol dipélargonate, la cyclopentadiméthylsiloxane, la cyclotétradiméthylsiloxane, le néopentanoate d'isostéaryle, le palmitate d'éthyle 2-héxyle, l'adipate de diéthylhéxyle, l'isostéarate d'isopropyle, le C₁₂-C₁₅ alkylbenzoate , le tétra-éthyl-2 héxanoate de pentaérythrityle, l'huile de macadamia, la néopentanoate d'isodécyle, la phényl triméthicone, le dioctanoate de propylène glycol, l'éthyle 2-hexanoate d'éthyle 2-héxyle, le diisopropyle dimer dilinoléate, l'éthyle 2-héxanoate de cétyle, le dicaprylyl maléate, l'isononanoate d'isononyle, la cyclohexadiméthylesiloxane, le citrate de triisocétyle, le dodécane di-oate de diisocétyle, le citrate de tri-iso arachidyle, le citrate de tri-isostéaryle, le néopentanoate d'octyldodécyle,le tri iso nonanoate de glycéryle, le tétraisostéarate de pentaérythrityle, le tri-mellilate de tri-décyle, le tri isotéarate de glycéryle, le di hepatnoate de néopentyl glycol, la tricapryline, le polyglycéryle-2 triisotéarate, le tridécyletétradécanoine, le copolymère dimères dilinoleyl diol / dimères dilinoléiques, le carylyl carbonate, l'hexyldecyl myristoyl methylaminopropionate , le polyglyceryl -10 nonaisostéarate , le dipentaerythrityl hexacaprylate/hexacaprate, le copolymère hydrogéné de dimer dilinoleyl / dimethylcarbonate, le di-caprate de néopentyl glycol, le tri-octanoate de glycéryle, l'iso-stéarate d'éhyl-2 héxyle, le diéthylène glycol di isononanoate, l'undécylpentadécanol, le benzoate d'isostéaryle, l'isononanoate d'isodécyle, adipate de di-isostéaryle, le tridécyl isononanoate, la triisopalmitine, l'hydroxy-stéarate d'octyl-2 dodécyle, Isostéarate de butyle, l'isononanoate de décyl-2 héxyle, le benzoate d'octyl-2 dodécyle, le dodécane-di-oate de di-iso-arachidyle, le di-iso-stéarate de propylene glycol, le tétra-isononanoate de pentaérythrityle, le tétrapélargonate de pentaérythrityle, le tétra-octyldodécanoate de pentaérythrityle, le tétra -décyltétradécanoate de pentaérythrityle, le polyglycéryl-2 tetraisostearate, le trimellitate de tri-iso-décyle, le trimellitate de tri-éthyl-2-héxyle, le trimellitate d'isofol 12, le tétra-2-hexyldécanoate de pentaérythrityle, le di-adipate de myristyle oxypropyléné (3OP), Benzoate d'ethyle-2 hexyle, tetraisostéarate de ditryméthylolpropane, le benzoate d'huile de ricin, le dioctyl carbonate (éthyl-2 hexyl), l'octyldodécyl PPG-3 myristyl éther dimer dilinoléate, triméthylolpropane triisostéarate, le sucrose 6-8 chaînes grasses soja, l'huile de sésame et leurs mélanges.

Selon un aspect de l'invention, l'huile ayant un paramètre de solubilité δₐ compris entre 0 et 7,00 (J/cm³)^{1/2} est une huile hydrocarbonée choisie parmi les alcanes aprotiques.
Selon un mode encore préféré, l'alcane aprotique présente une masse moléculaire allant de 165 à 900 g/mol, et plus préférentiellement de 250 à 800 g/mol
Les alcanes aprotiques préférés au sens de la présente demande peuvent en particulier être choisis parmi les polyisobutylène de poids moléculaire compris entre 250 et 800 g/mol, le squalane, l'huile de parléam et leurs mélanges.

Selon un autre aspect de l'invention, l'huile ayant un paramètre de solubilité δₐ compris entre 0 et 7,00 (J/cm³)^{1/2} est une huile siliconée.

Les huiles siliconées ayant un paramètre de solubilité δₐ compris entre 0 et 7,00 (J/cm³)^{1/2} peuvent notamment être choisies parmi les huiles de silicone linéaires telles que les polydiméthylsiloxanes, la cyclopentadiméthylsiloxane, la cyclotétradiméthylsiloxane, la phényl triméthicone, la cyclohexadiméthylesiloxane et leur mélange.

De préférence, huile siliconée est ramifiée par des groupements de faible poids moléculaire, notamment en C₁-C₃, tel que des groupements méthyle ou éthyle.

L'huile siliconée ayant un paramètre de solubilité δₐ compris entre 0 et 7,00 (J/cm³)^{1/2} présente de préférence un poids moléculaire compris entre 100 et 800 g/mol, et plus préférentiellement de 300 à 500 g/mol.

L'huile ayant un paramètre de solubilité δₐ compris entre 0 et 7,00 (J/cm³)^{½} peut être présente dans la composition selon l'invention en une teneur allant de 20 à 100% en poids, par rapport au poids total de la phase grasse liquide continue, de préférence de 40 à 99% en poids, et mieux encore de 60 à 95% en poids.

### Huiles additionnelles :

Outre les huiles particulières précédemment définies, la composition selon l'invention peut comprendre au moins une huile additionnelle choisie parmi les huiles volatiles, les huiles non volatiles et leur mélange.

Selon un mode préféré de réalisation, la composition selon l'invention peut comprendre au moins une huile volatile

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

Selon un mode préféré de réalisation, la composition selon l'invention peut comprendre au moins une huile volatile hydrocarbonée.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

La composition selon l'invention peut comprendre une huile volatile en une teneur allant de 1 à 50 % en poids par rapport au poids total de la composition, de préférence de 2 à 30 % en poids, plus préférentiellement allant de 3 à 15 % en poids..

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

L'huile non volatile peut être présente dans la composition selon l'invention en une teneur allant de 1 % à 50 % en poids, par rapport au poids total de la composition, de préférence allant de 2 % à 30 % en poids, de préférence allant de 3 % à 20 % en poids.

La phase grasse liquide peut être présente dans la composition selon l'invention en une teneur totale allant de 10 à 95 % en poids par rapport au poids total de la composition, de préférence de 20 à 90 % en poids, plus préférentiellement allant de 30 à 85 % en poids.

### Corps gras

La composition selon l'invention peut également comprendre des corps gras autres que les huiles citées ci-dessus, comme des cires ou encore des corps gras pâteux.

Par cires on entend un corps gras solide à température ambiante.

On peut définir les corps gras pâteux à l'aide d'au moins une des propriétés physico-chimiques suivantes :
- une viscosité de 0,1 à 40 Pa.s (1 à 400 poises), mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

A titre de cires pouvant être utilisées selon l'invention, on peut citer :
- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la C₃₀-C₄₅ alkyl méthicone vendue sous la dénomination commerciale "AMS C 30" par DOW CORNING,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale "KESTER WAX K82H" par la société KOSTER KEUNEN,
- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.

Sans être limité par une quelconque théorie, les cires peuvent permettre de renforcer les propriétés de cicatrisation et diminuer le caractère collant à pâteux des compositions selon l'invention.

De préférence, les cires sont présentes à une teneur allant de 0,1 à 30% en poids, de préférence encore de 0,5 à 20 % en poids, par rapport au poids total de la composition.

Ces corps gras peuvent en particulier être choisis de manière variée par l'homme du métier afin de préparer une composition ayant les propriétés souhaitées, par exemple en consistance ou en texture.

### Agent épaississant additionnel

Outre le composé de bis-urée précédemment décrit, la composition selon l'invention peut comprendre, au moins un agent épaississant additionnel des huiles choisi parmi les agents épaississants polymériques, les agents épaississants minéraux et leurs mélanges.

L'agent épaississant polymérique des huiles est capable d'épaissir ou de gélifier la phase organique de la composition. L'agent épaississant polymérique peut être également filmogène, c'est-à-dire qu'il est capable de former un film lors de son application sur la peau. L'agent épaississant polymérique des huiles peut notamment être choisi parmi:
- les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG^{®} par la société ARIZONA CHEMICAL ;
- les polymères siliconés du type :
   1) des polyorganosiloxanes comportant au moins deux groupes capables d'établir des interactions hydrogène, ces deux groupes étant situés dans la chaîne du polymère, et/ou
   2) des polyorganosiloxanes comportant au moins deux groupes capables d'établir des interactions hydrogène, ces deux groupes étant situés sur des greffons ou ramifications. Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.

Les polymères siliconés utilisés comme agents structurants dans la composition de l'invention sont des polymères du type polyorganosiloxane comme par exemple ceux décrits dans les documents US-A-5 874 069, US-A-5,919,441, US-A-6,051,216 et US-A-5,981,680.
En particulier, les polymères siliconés sont des polyorganosiloxanes tels que définis ci-dessus et dont les motifs capables d'établir des interactions hydrogènes sont disposés dans la chaîne du polymère.
Les polymères siliconés peuvent plus particulièrement être des polymères comprenant au moins un motif répondant à la formule générale 1 : 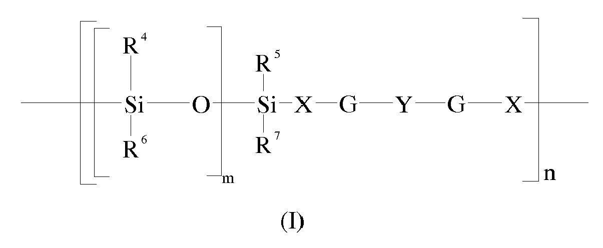 dans laquelle :
1) R⁴, R⁵, R⁶ et R⁷, identiques ou différents, représentent un groupe choisi parmi :
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote,
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 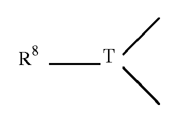 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
   - R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi : 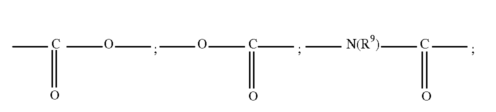 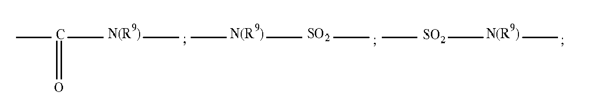 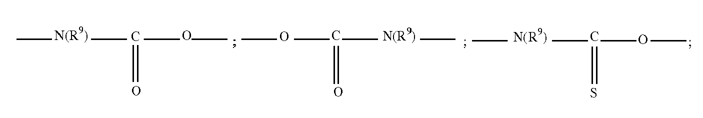 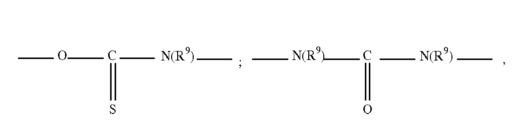 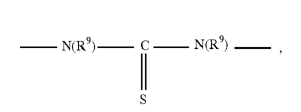 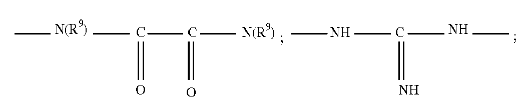 et 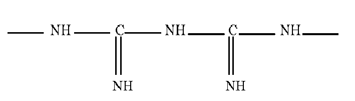 où R⁹ représente un atome d'hydrogène ou un groupe alkyle, linéaire ou ramifié, en C₁ à C₂₀, à condition qu'au moins 50 % des R⁹ du polymère représente un atome d'hydrogène et qu'au moins deux des groupes G du polymère soient un autre groupe que : 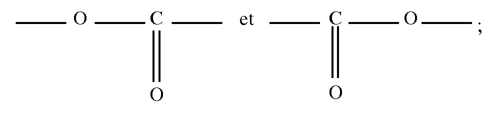
6) n est un nombre entier allant de 2 à 500, de préférence de 2 à 200, et m est un nombre entier allant de 1 à 1000, de préférence de 1 à 700 et mieux encore de 6 à 200.
   Selon l'invention, 80 % des R⁴, R⁵, R⁶ et R⁷, du polymère sont choisis de préférence parmi les groupes méthyle, éthyle, phényle et 3,3,3-trifluoropropyle.
   Selon un mode de réalisation avantageux, les groupes capables d'établir des interactions hydrogènes sont des groupes amides de formule -C(O)NH- et -HN-C(O)-.
   Dans ce cas, l'agent structurant peut être un polymère comprenant au moins un motif de formule (III) ou (IV) : 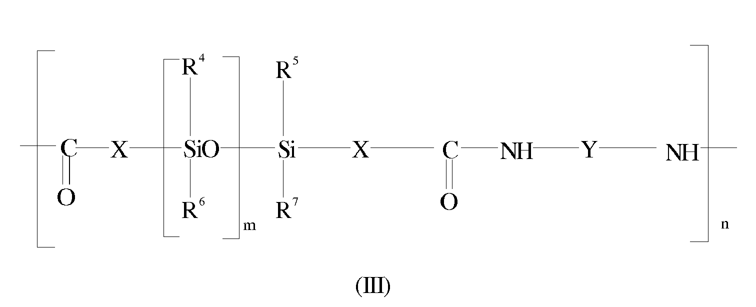
ou 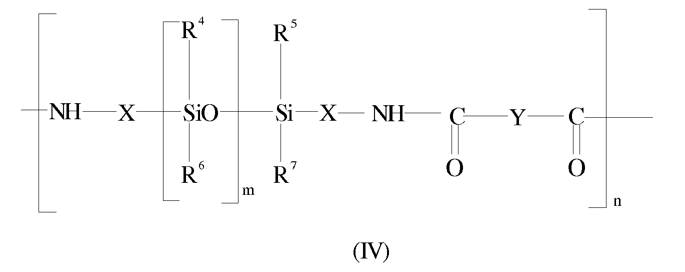 dans lesquelles R⁴, R⁵, R⁶, R⁷, X, Y, m et n sont tels que définis ci-dessus.
Dans ces polyamides de formule (III) ou (IV), m va de 1 à 700, en particulier de 15 à 500 et notamment de 50 à 200 et n va particulier de 1 à 500, de préférence de 1 à 100 et mieux encore de 4 à 25,
- X est de préférence une chaîne alkylène linéaire ou ramifiée ayant de 1 à 30 atomes de carbone, en particulier 1 à 20 atomes de carbone, notamment de 5 à 15 atomes de carbone et plus particulièrement de 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.
- les galactomananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges ;

La composition selon l'invention peut également comprendre au moins un agent épaississant minéral des huiles tel qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.
Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques comme la peau.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Bentone ISD V par la société Elementis, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

Sans être limité par une quelconque théorie, les agents épaississants minéraux peuvent permettre de renforcer les propriétés de cicatrisation et diminuer le caractère collant à pâteux des compositions selon l'invention.

L'agent épaississant additionnel des huiles peut être présent dans la composition selon l'invention en une teneur en matière active allant de 0,01 % à 15 % en poids, par rapport au poids total de la composition, de préférence en une teneur allant de 0 ,1 % à 10 % en poids, et préférentiellement allant de 0,3 % à 5 % en poids.

### Phase aqueuse

La composition selon l'invention peut comprendre une phase aqueuse.

La phase aqueuse comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants organiques miscibles à l'eau (à température ambiante - 25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ; les éthers de glycol (ayant notamment de 3 à 16 atomes de carbone) tels que les alkyl(C1-C4)éther de mono, di- ou tripropylène glycol, les alkyl(C1-C4)éthers de mono, di- ou triéthylène glycol, et leurs mélanges.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

De préférence, la phase aqueuse peut être présente dans la composition selon l'invention en une teneur allant de 1 à 80 % en poids, par rapport au poids total de la composition, et de préférence de 5 à 50 % en poids, et plus préférentiellement de 5 à 40 % en poids.
Selon un mode de réalisation, la composition selon l'invention peut également être anhydre. Par composition anhydre au sens de la présente demande, on entend une composition comprenant moins de 5 % en poids d'eau, par rapport au poids total de la composition, de préférence moins de 2 % en poids d'eau. Plus préférentiellement encore, la composition est exempte d'eau, l'eau n'étant pas ajoutée lors de la préparation de la composition mais correspondant à l'eau résiduelle apportée par les ingrédients mélangés.

Selon un mode particulier de réalisation, la phase aqueuse et la phase huileuse peuvent être présente en une teneur telle que le rapport pondéral d'eau sur huile est supérieur ou égal à 1 et notamment compris entre 1 et 1,2.

### Phase pulvérulente

La composition selon l'invention peut comprendre une phase pulvérulente comprenant notamment des pigments, des charges, des nacres et/ou leurs mélanges.

Selon un mode préféré de réalisation, la composition selon l'invention peut comprendre des pigments.

Par « pigments », il faut comprendre des particules, minérales ou organiques, insolubles dans la phase organique liquide, destinées à colorer et/ou opacifier la composition.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique et leurs mélanges.
On utilise de préférence des pigments d'oxydes de fer et/ou de dioxyde de titane.

Les pigments peuvent être traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine.

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1 086683.

Les pigments peuvent être présents, dans la composition selon l'invention, en une teneur allant de 0,1 à 40% en poids, par rapport au poids total de la composition, en particulier allant de 1% à 30% en poids, et plus préférentiellement allant de 5 % à 15% en poids.

Outre les pigments, la phase pulvérulente de la composition selon l'invention peut comprendre des charges et/ou des nacres.

Selon un mode préféré de réalisation, la composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®), les poudres de poly-β-alanine, les poudres de polyéthylène, les polyméthacrylates de méthyle, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), les particules de cire micronisée notamment les microcires de Carnauba telles que celles commercialisées sous la dénomination de « MicroCare 350^{®} » par la société MICRO POWDERS, les microcires de cire synthétique telles que celles commercialisées sous la dénomination de « MicroEase 114S^{®} » par la société MICRO POWDERS, les microcires constituées d'un mélange de cire de Carnauba et de cire de polyéthylène telles que celles commercialisées sous les dénominations de « MicroCare 300^{®} » et « 310^{®} » par la société MICRO POWDERS, les microcires constituées d'un mélange de cire de Carnauba et de cire synthétique telles que celles commercialisées sous la dénomination de « MicroCare 325^{®} » par la société MICRO POWDERS, les microcires de polyéthylène telles que celles commercialisées sous les dénominations de « MicroPoly 200^{®} », « 220^{®} », « 220L^{®}», et « 250S^{®} », par la société MICRO POWDERS, et celles commercialisées sous la dénomination de « CERAPURE H5-C » par la société SHAMROCK, ou les microcires de polypropylène telle que celles commercialisées sous la dénomination « MATTEWAX » par la société MICRO POWDERS ; la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Selon un mode préféré de réalisation, la composition selon l'invention peut comprendre une poudre de polytétrafluoroéthylène (PTFE).

Les charges peuvent être présentes dans la composition selon l'invention en une teneur totale allant de 0,1 % à 30 % en poids, par rapport au poids total de la composition, de préférence allant de 0,5 % à 20 % en poids, et préférentiellement allant de 0,8 % à 10 % en poids.

Outre les pigments et les charges, la phase particulaire de la composition selon l'invention peut comprendre des nacres.

Par « nacres », il faut comprendre des particules irisées, notamment produites par certains mollusques dans leur coquille ou bien synthétisées, insolubles dans le milieu de la composition.

Les nacres peuvent être choisies parmi les pigments nacrés blancs tels que l'oxychlorure de bismuth, le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

### Colorants additionnels

La composition selon l'invention peut comprendre des colorants additionnels choisis parmi les colorants hydrosolubles et liposolubles.

Les colorants hydrosolubles sont par exemple le jus de betterave, le bleu de méthylène, le caramel.

Par colorants liposolubles, il faut comprendre des composés généralement organiques solubles dans les corps gras comme les huiles.
Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n°2, le D&C orange n°5, le jaune quinoléine, le rocou, les bromoacides.

### Galéniques

La composition selon l'invention peut se présenter sous différentes formes galéniques, notamment anhydre ou émulsions eau-dans-huile, ou multiples à phase continue huileuse, et se présenter sous forme de gel, crèmes, suspensions, compact, coulés à chaud et sticks.

La composition selon l'invention peut se présenter sous forme d'une composition de maquillage, notamment un produit pour le teint tel qu'un fond de teint, un fard à joues ou à paupières; un produit pour les lèvres tel qu'un rouge à lèvres, un gloss ou un soin des lèvres; un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps ou des cheveux (mascara ou laque pour cheveux); d'une composition de protection ou de soin de la peau du visage, du cou, des mains ou du corps, notamment une composition anti-rides, anti-fatigue, anti-âge, permettant de donner un coup d'éclat à la peau, une composition hydratante ou traitante; une composition anti-solaire ou après-soleil ou de bronzage artificiel; d'une composition capillaire, notamment pour la coloration, le soin, l'hygiène des cheveux, le coiffage, le maintien de la coiffure ou la mise en forme des cheveux.

### Ingrédients cosmétiques usuels additionnels

La composition selon l'invention peut comprendre au moins un autre ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les sequestrants, les agents filmogènes, et leurs mélanges.

L'invention est présentée plus en détail dans les exemples ci-après.

### Exemple 1

On a préparé un fond de teint ayant la composition suivante :

| **PHASE** | **Nom** | **Concentration** |
|---|---|---|
| | | **(% massique)** |
| A1 | Composé de formule générale (I) sous forme de mélange majoritairement constitué des composés : | |
| | 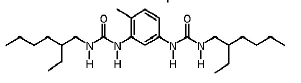 | |
| | 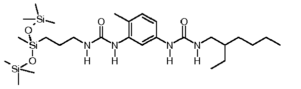 | 1,50 |
| | et | |
| | 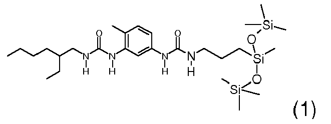 | |
| | Huile de Parléam | 32,10 |
| A2 | Cétyl diméthicone copolyol (2) | 1,80 |
| | Mono/diglycérides d'acides isostéarique estérifié par l'acide succinique (3) | 0,60 |
| A3 | Cyclopentasiloxane | 5,00 |
| | Pigments d'oxydes de fer | 2,04 |
| | Pigments de dioxyde de titane | 7,96 |
| A4 | Nylon 12 | 8,00 |
| B | Eau | 40,00 |
| | Conservateurs | 1,00 |
| | TOTAL | 100,000 |

| | | |
|---|---|---|
| (1) La préparation du mélange (1) est décrite dans le mode opératoire ci-après. (2) Vendu sous la dénomination Abil EM 90 par la Société Goldschmidt. (3) Vendu sous la dénomination Imwitor 780 k par la Société Sasol. | | |

### Mode opératoire :

### 1. Préparation du composé de formule générale (I)

On a effectué la préparation de composés de type bis-urées, comportant au moins un motif siliconé et au moins un motif non siliconé.
Le mélange est obtenu en une étape, le schéma de synthèse étant représenté ci-dessous :

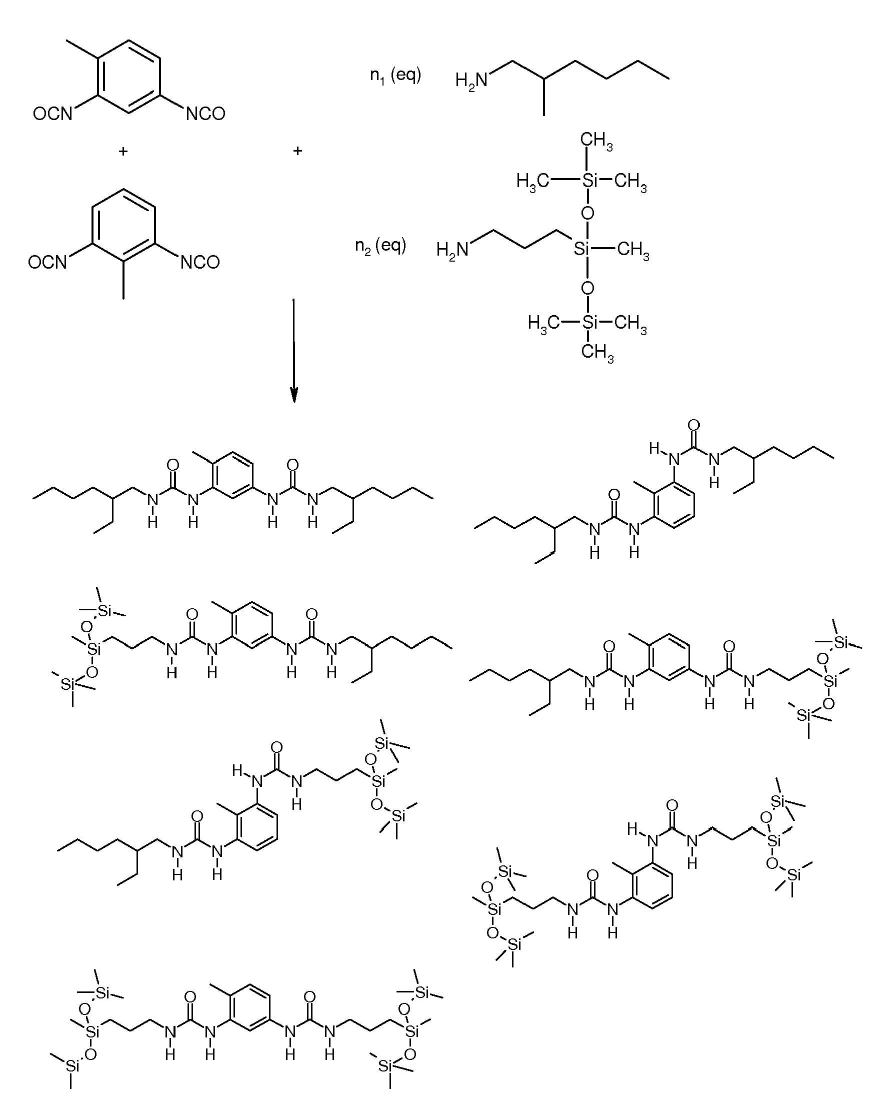

Les matières premières de départ sont les suivantes :
- Diisocyanate de toluilène (TDI) : 95/5 en (isomère 2,4)/(isomère 2,6); m=50 g (0,29 moles)
- n₁= 0,44 moles (1,5 éq.) soit m=56,87 g de 2-éthylhexylamine;
- n₂= 0,19 moles (0,66 éq.) soit m= 53,13 g de 3-aminopropylméthylbis-(triméthylsiloxy)silane, avec un taux d'isomère bêta inférieur à 1%.
On a donc n_{R}/n'_{R}= 2,23 (69/31).

On mélange le diisocyanate de toluilène en solution dans le THF anhydre avec 2,2 équivalents d'amine en solution dans le THF anhydre.
La réaction est effectuée sous atmosphère inerte (argon) en milieu anhydre avec une température du milieu réactionnel qui est maintenue entre 15°C et 40°C.
Parallèlement, on prépare une solution d'amine (Y) dans le THF. La température du milieu réactionnel ne devant pas, de préférence, dépasser 40°C, la concentration de l'amine et la vitesse d'addition de la solution d'amine (Y) sont ajustées à cette nécessité. On laisse le milieu réactionnel sous agitation en contrôlant l'avancement de la réaction par spectrométrie infra-rouge (disparition de la bande NCO entre 2250 et 2280 cm⁻¹).
Une fois que le diisocyanate a complètement réagi, le mélange réactionnel est additionné à de l'eau acidifiée (pH 3) avec de l'acide chlorhydrique, le précipité obtenu est filtré, lavé plusieurs fois à l'eau et enfin séché sous vide ou lyophilisé. Une poudre blanche est obtenue et utilisée telle quelle après analyse (HPLC couplée à la masse).

Le mélange obtenu au final comprend les bis-urées suivantes:

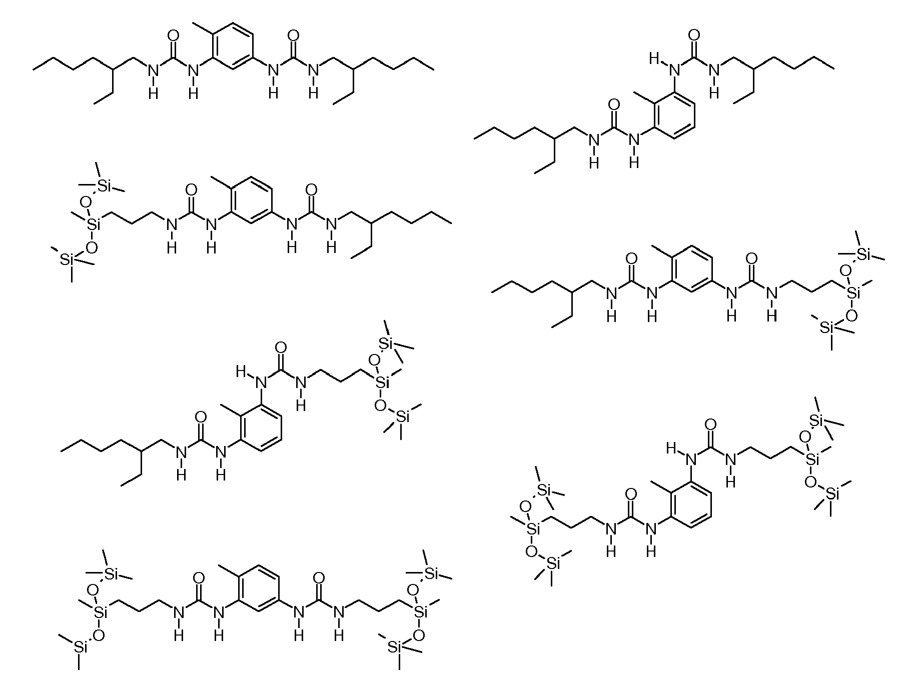

### 2. Préparation de la composition de fond de teint

On fait fondre dans un bécher, à une température de 100°C, les composés de la phase A1 sous agitation magnétique pendant environ une heure (phase A1).

On ajoute à la phase A1 les composés de la phase A2 sous agitation au Moritz, à une température de 80°C (Phases A1 + A2).

On broie à la tricylindre les pigments de la phase A3 dans la cyclopentasiloxane, puis on ajoute la phase A3 obtenue au mélange A1 + A2.

On ajouter toujours sous agitation la poudre de nylon de la phase A4 en la saupoudrant sur le mélange A1 +A2+A3.

Dans un bécher séparé, on pèse l'eau et les conservateurs. On place ce bécher sur une plaque chauffante et sous agitation magnétique afin de dissoudre les conservateurs dans l'eau. On laisse revenir le mélange B à température ambiante, puis on le verse doucement dans le mélange A1 + A2 + A3 + A4 pour former l'émulsion.

Le fond de teint obtenu présente une texture crémeuse qui, une fois appliquée sur la peau, forme un film lisse. En outre, après la prise au doigt du produit dans le pot (ou cisaillement manuel), la surface du produit dans le pot reprend sa forme initiale (cicatrisation de la surface).

### Exemple 2

On a préparé un fond de teint ayant la composition suivante :

| **PHASE** | **Nom** | **Concentration** |
|---|---|---|
| | | **(% massique)** |
| A1 | Composé de formule générale (1) sous forme de mélange majoritairement constitué des composés : | |
| | 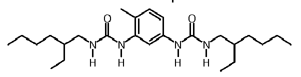 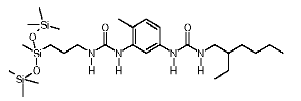 | 2,50 |
| | et | |
| | 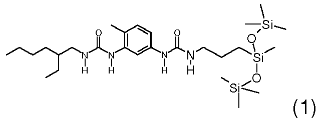 | |
| | Huile de Parléam | 31,10 |
| A2 | Cétyl diméthicone copolyol (2) | 1,80 |
| | Mono/diglycérides d'acides isostéarique estérifié par l'acide succinique (3) | 0,60 |
| A3 | Cyclopentasiloxane | 5,00 |
| | Pigments d'oxydes de fer | 2,04 |
| | Pigments de dioxyde de titane | 7,96 |
| A4 | Nylon 12 | 8,00 |
| B | Eau | 40,00 |
| | Conservateurs | 1,00 |
| | TOTAL | 100,000 |

| | | |
|---|---|---|
| (1) La préparation du mélange (I) est effectuée selon le même protocole que l'exemple 1. (2) Vendu sous la dénomination Abil EM 90 par la Société Goldschmidt. (3) Vendu sous la dénomination Imwitor 780 k par la Société Sasol. | | |

Le mode opératoire pour obtenir le fond de teint est également le même que celui de l'exemple 1. Le fond de teint obtenu présente une texture crémeuse qui, une fois appliquée sur la peau, forme un film lisse. En outre, après la prise au doigt du produit dans le pot (ou cisaillement manuel), la surface du produit dans le pot reprend sa forme initiale (cicatrisation de la surface).

### Exemple 3

On a préparé un fond de teint ayant la composition suivante :

| **PHASE** | **Nom** | **Concentration** |
|---|---|---|
| | | **(% massique)** |
| A1 | Composé de formule générale (I) sous forme de mélange majoritairement constitué des composés : | |
| | 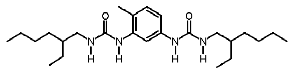 | |
| | 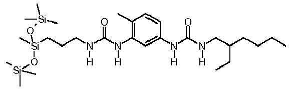 | 1,50 |
| | et | |
| | 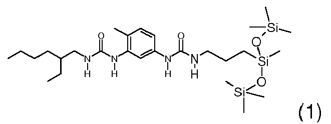 | |
| | Cyclopentasiloxane | 32,10 |
| A2 | Cétyl diméthicone copolyol (2) | 1,80 |
| | Mono/diglycérides d'acides isostéarique estérifié par l'acide succinique (3) | 0,60 |
| A3 | Cyclopentasiloxane | 5,00 |
| | Pigments d'oxydes de fer | 2,04 |
| | Pigments de dioxyde de titane | 7,96 |
| A4 | Nylon 12 | 8,00 |
| B | Eau | 40,00 |
| | Conservateurs | 1,00 |
| | TOTAL | 100,000 |

| | | |
|---|---|---|
| (1) La préparation du mélange (1) est effectuée selon le même protocole que l'exemple 1. (2) Vendu sous la dénomination Abil EM 90 par la Société Goldschmidt. (3) Vendu sous la dénomination Imwitor 780 k par la Société Sasol. | | |

Le mode opératoire pour obtenir le fond de teint est également le même que celui de l'exemple 1. Le fond de teint obtenu présente une texture crémeuse qui, une fois appliquée sur la peau, forme un film lisse. En outre, après la prise au doigt du produit dans le pot (ou cisaillement manuel), la surface du produit dans le pot reprend sa forme initiale (cicatrisation de la surface).

### Exemple 4

On a préparé un fond de teint ayant la composition suivante :

| **PHASE** | **Nom** | **Concentration** |
|---|---|---|
| | | **(% massique)** |
| A1 | Composé de formule générale (1) sous forme de mélange majoritairement constitué des composés : | |
| | 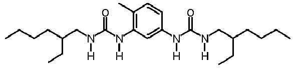 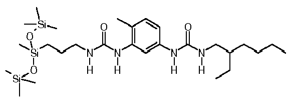 | 2,50 |
| | et | |
| | 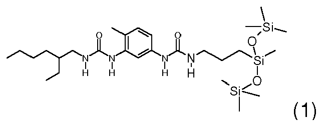 | |
| | Cyclopentasiloxane | 31,10 |
| A2 | Cétyl diméthicone copolyol (2) | 1,80 |
| | Mono/diglycérides d'acides isostéarique estérifié par l'acide succinique (3) | 0,60 |
| A3 | Cyclopentasiloxane | 5,00 |
| | Pigments d'oxydes de fer | 2,04 |
| | Pigments de dioxyde de titane | 7,96 |
| A4 | Nylon 12 | 8,00 |
| B | Eau | 40,00 |
| | Conservateurs | 1,00 |
| | TOTAL | 100,000 |

| | | |
|---|---|---|
| (1) La préparation du mélange (I) est effectuée selon le même protocole que l'exemple 1. (2) Vendu sous la dénomination Abil EM 90 par la Société Goldschmidt. (3) Vendu sous la dénomination Imwitor 780 k par la Société Sasol. | | |

Le mode opératoire pour obtenir le fond de teint est également le même que celui de l'exemple 1. Le fond de teint obtenu présente une texture crémeuse qui, une fois appliquée sur la peau, forme un film lisse. En outre, après la prise au doigt du produit dans le pot (ou cisaillement manuel), la surface du produit dans le pot reprend sa forme initiale (cicatrisation de la surface).

## Revendications

1. Composition cosmétique comprenant au moins une phase grasse liquide continue, comprenant au moins un composé de formule générale (I), ou l'un de ses sels et/ou isomères : 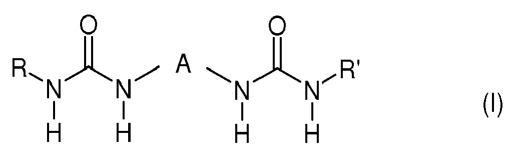 dans laquelle :
- A est un groupement de formule (II) : 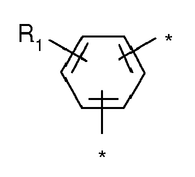 avec R1 étant un radical alkyle C₁ à C₄ linéaire ou ramifié, et les * symbolisant les points de rattachement du groupement A à chacun des deux atomes d'azote du reste du composé de formule générale (1), et
- R et R', identiques ou différents, sont choisis parmi :
- i) les radicaux de formule (III) : 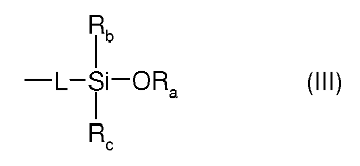 dans laquelle :
- L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, 0 et S;
- Ra est :
a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
b) un radical siliconé de formule : 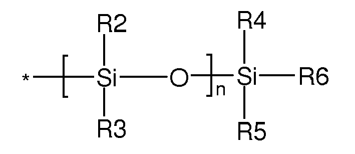 avec n étant compris entre 0 et 100, notamment entre 1 et 80, voire 2 à 20;
et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;
- Rb et Rc sont, indépendamment l'un de l'autre, choisis parmi:
a) les radicaux carbonés, notamment hydrocarbonés (alkyles), linéaires, ramifiés et/ou cycliques, saturés
ou insaturés, comprenant 1 à 18 atomes de carbone, et pouvant comprendre 1 à 4 hétéroatomes choisis parmi N, O, Si et S;
b) les radicaux de formule : 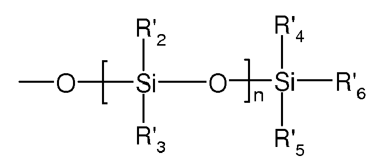 avec n étant compris entre 0 et 100, notamment entre 1 et 80, voire 2 à 20;
et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
et
- ii) les radicaux alkyle en C₁ à C₃₀, linéaire, ramifié et/ou cyclique, saturé ou insaturé, et comprenant éventuellement 1 à 3 hétéroatomes choisis parmi O, S, F et N;
étant entendu que l'un au moins des radicaux R et/ou R' est de formule (III) ; et
au moins une huile ayant un paramètre de solubilité δₐ compris entre 0 et 7,00 (J/cm³)^{½}, et de préférence entre 0 et 5,00 (J/cm³)^{½}.

2. Composition selon la revendication 1, dans laquelle le groupement A est de formule : 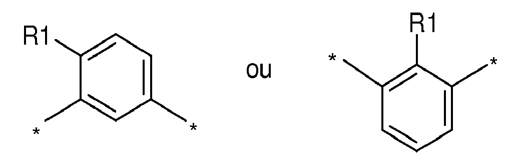 avec R1 et les * étant tels que définis dans la revendication 1.

3. Composition selon l'une des revendications précédentes, dans laquelle le groupement A est de formule :

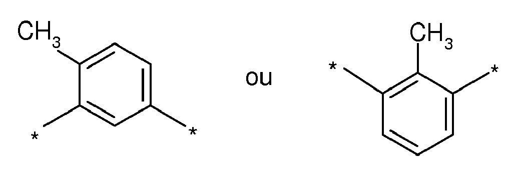

4. Composition selon l'une des revendications précédentes, dans laquelle L est de structure -(CH₂)n- avec n= 1 à 18, notamment 2 à 12, voire 3 à 8; notamment méthylène, éthylène, propylène, butylène, ou octylène; ou bien L est de structure -CH₂-CH(CH₃)-.

5. Composition selon l'une des revendications précédentes, dans laquelle le radical Ra est choisi parmi les radicaux :
- de structure -(CH₂)n'-CH₃ avec n'= 0 à 17, notamment 1 à 12, voire de 1 à 6; en particulier, méthyle, éthyle, propyle ou butyle; ou bien
- de structure -(CH₂)x-O-(CH₂)z-CH₃ ou bien -(CH₂)x-O-(CH₂)y-O-(CH₂)z-CH₃, avec x = 1 à 10, de préférence 2; y=1 à 10 de préférence 2, et z= 0 à 10, de préférence 0 ou 1 ; ou bien
- de structure -SiR₄R₅R₆, dans laquelle R4, R5 et R6 sont, indépendamment les uns des autres, des radicaux alkyle ayant 1 à 12 atomes de carbone, notamment 1 à 6 atomes de carbone; en particulier R4, R5 et/ou R6 sont méthyle, éthyle, propyle, butyle; ou bien
- de formule : 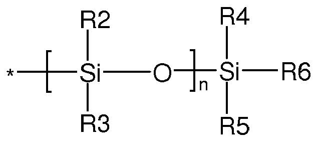
dans laquelle R2 à R6 sont, indépendamment les uns des autres, des radicaux alkyle ayant 1 à 12 atomes de carbone, notamment 1 à 6 atomes de carbone; en particulier R2 à R6 sont méthyle, éthyle, propyle, butyle.

6. Composition selon l'une des revendications précédentes, dans laquelle Ra est de structure : 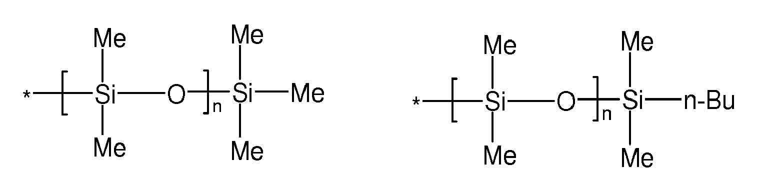 avec n=1 à 100; et encore plus particulièrement un radical :

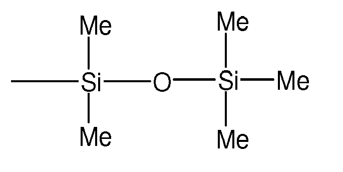

7. Composition selon l'une des revendications précédentes, dans laquelle les radicaux Rb et Rc, identiques ou différents, sont choisis parmi les radicaux :
- de structure -(CH₂)m-CH₃ avec m= 0 à 17, notamment 1 à 12, voire 2 à 5; en particulier, méthyl, éthyle, propyle ou butyle; ou bien
- de structure -O-(CH₂)m'-CH₃ avec m'= 0 à 5, notamment 1 à 4, et en particulier méthoxy ou éthoxy; ou bien
- de structure -O-(CH₂)x-O-(CH₂)z-CH₃ ou -O-(CH₂)x-O-(CH₂)y-O-(CH₂)z-CH₃, avec x = 1 à 10, de préférence 2; y=1 à 10 de préférence 2, et z= 0 à 10, de préférence 0 ou 1 ; ou bien
- de structure : 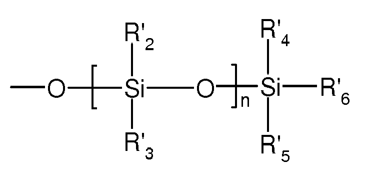 avec n étant compris entre 0 et 100, notamment entre 1 et 80, voire 2 à 20;
et R'2 à R'6 étant, indépendamment les uns des autres, de préférence, des radicaux alkyle, ayant 1 à 12 atomes de carbone, notamment 1 à 6 atomes de carbone; en particulier méthyle, éthyle, propyle, butyle.

8. Composition selon l'une des revendications précédentes, dans laquelle l'un au moins des radicaux R et/ou R' est choisi parmi les radicaux suivants : 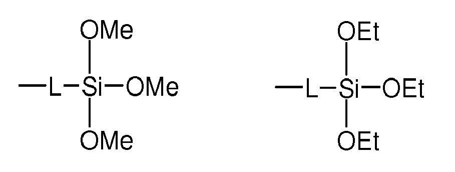 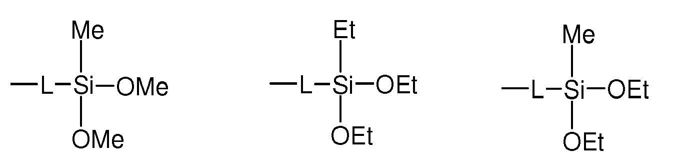 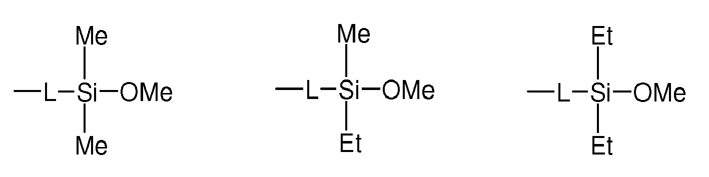 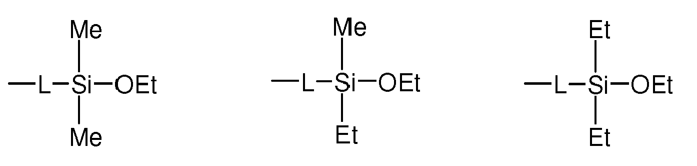 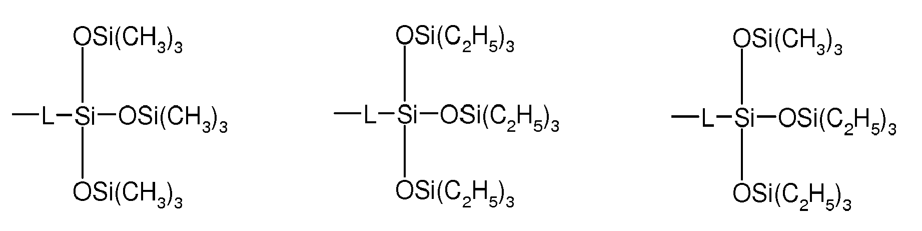 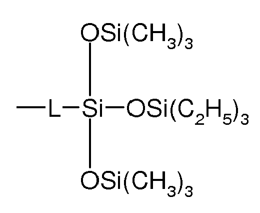 et également ceux de formule : 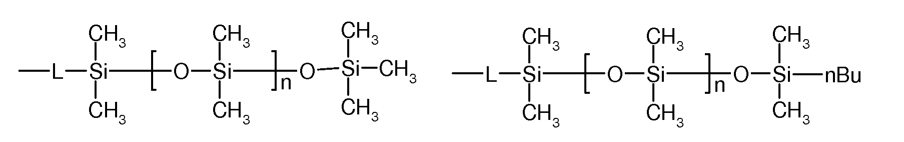 avec n variant de 0 à 100 et en particulier 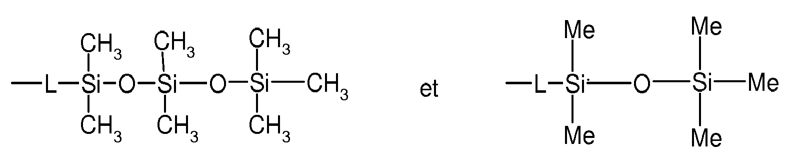 et 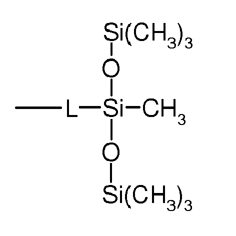 ou encore 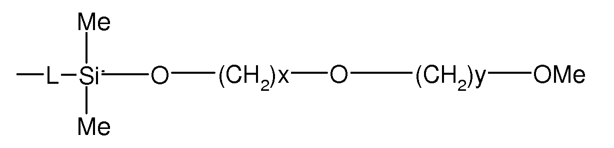 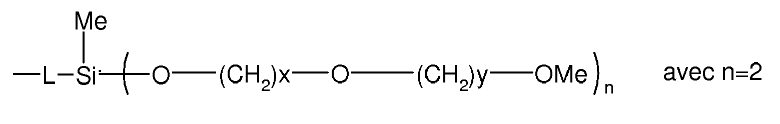
-L-Si(̵O-(CH₂)x-O-(CH₂)y-OMe)ₘ avec m=3
dans lesquelles x = 1 à 10, de préférence 2; et y = 1 à 10, de préférence 2;
et L étant tel que défini ci-dessus.

9. Composition selon la revendication 8, dans laquelle L est un radical alkylène en C1-C8, linéaire ou ramifié, notamment méthylène, éthylène, propylène, butylène et notamment n-butylène, octylène ou de formule -CH₂-CH(CH₃)-.

10. Composition selon l'une des revendications précédentes, dans laquelle R et R', identiques ou différents, sont tous les deux de formule (III).

11. Composition selon l'une des revendications 1 à 9, dans laquelle l'un des radicaux R ou R' représente un radical alkyle en C₁ à C₃₀, linéaire, ramifié et/ou cyclique, saturé ou insaturé, et comprenant éventuellement 1 à 3 hétéroatomes choisis parmi 0, S, F et N.

12. Composition selon la revendication 11, dans laquelle le radical R ou R' est choisi parmi : 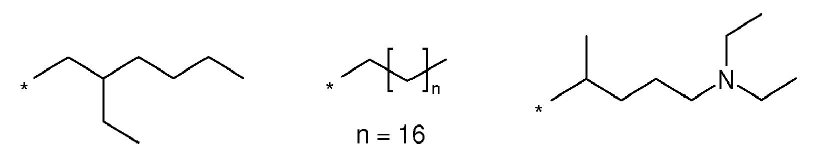 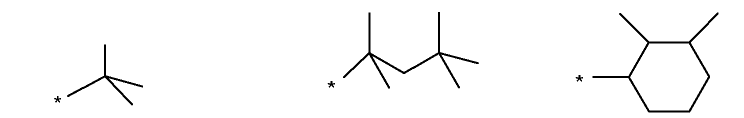 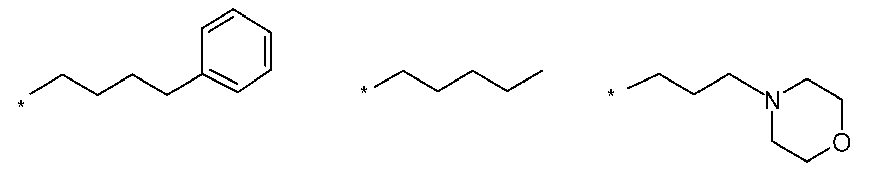 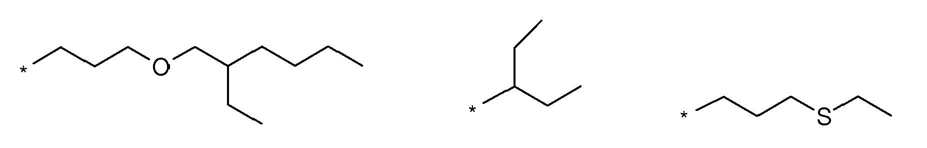 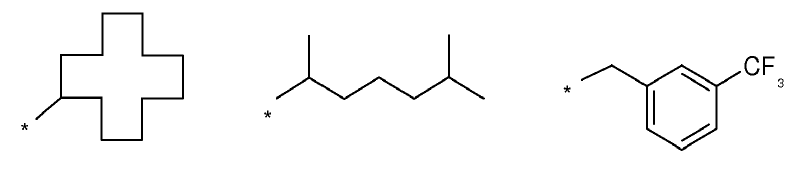 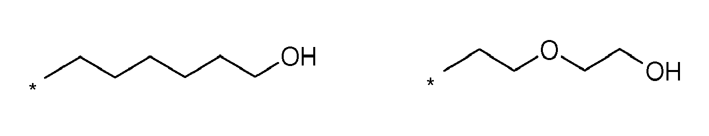 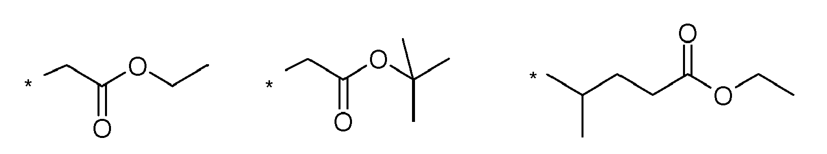 avec * ayant la définition donnée ci-dessus.

13. Composition selon l'une des revendications 1 à 9, dans laquelle le radical R ou R' représente un radical alkyle ramifié, notamment monoramifié, de préférence non cyclique, saturé ou insaturé, comprenant 3 à 16 atomes de carbone, notamment 4 à 12, voire 4 à 8 atomes de carbone, et comprenant éventuellement 1 à 3 hétéroatomes choisis parmi O, S, F et/ou N, de préférence O et/ou N.

14. Composition selon la revendication 13, dans laquelle le radical R ou R' est choisi parmi les radicaux tertio-butyle, 2-éthylhexyle ou de formule :

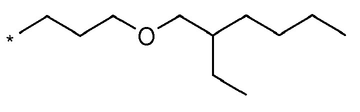

15. Composition selon l'une des revendications précédentes, dans laquelle le composé de formule (I) est choisi parmi, seul ou en mélange, les composés suivants, ainsi que leurs sels et isomères :

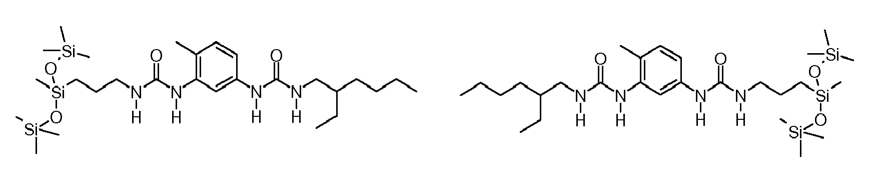

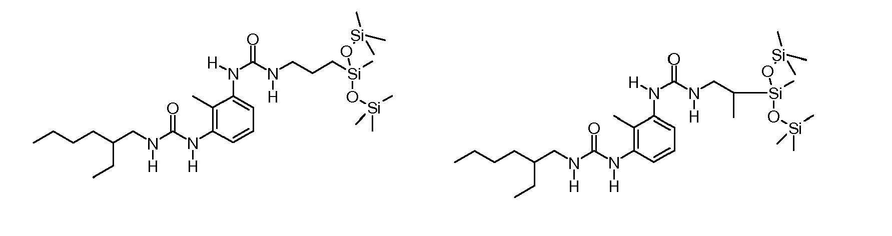

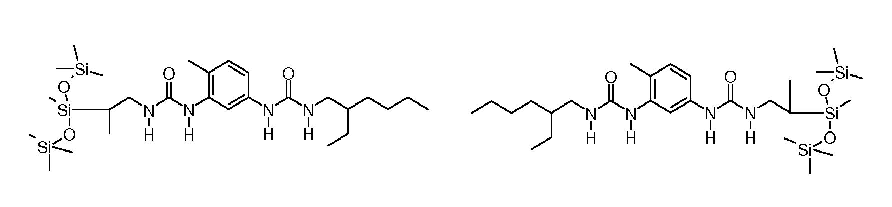

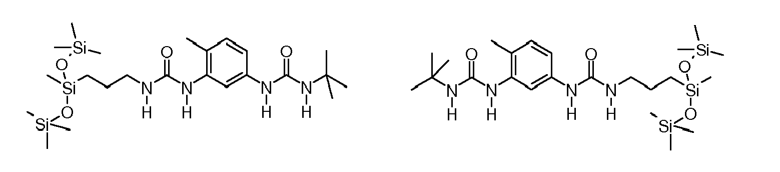

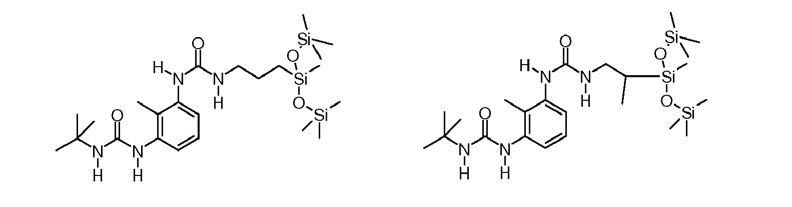

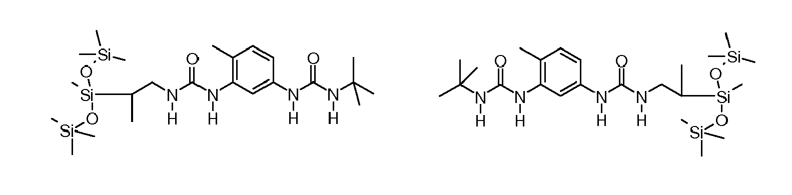

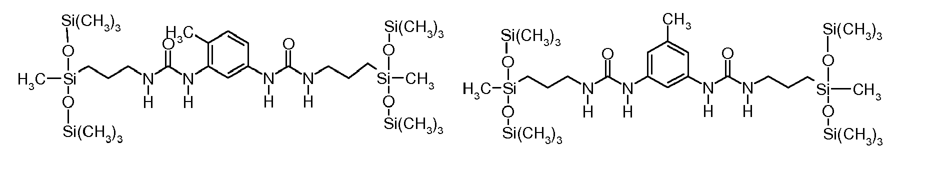

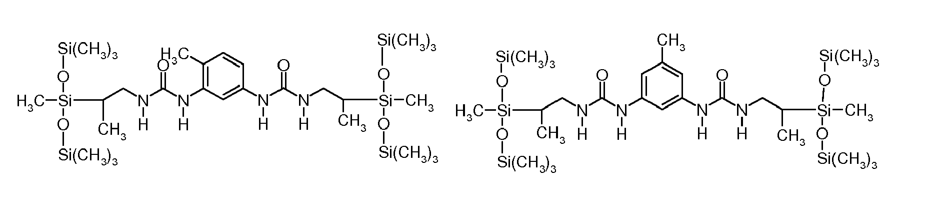

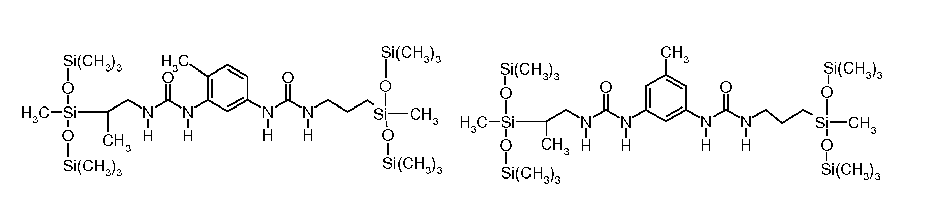

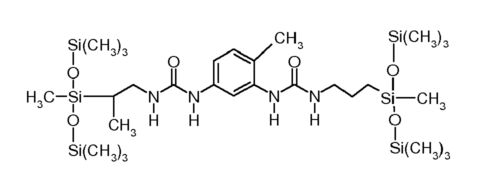

16. Composition selon l'une des revendications précédentes, comprenant de 0,01 à 20% en poids, par rapport au poids total de la composition, de composé(s) de formule (I), notamment de 0,05 à 15% en poids, voire de 0,1 à 10% en poids, mieux de 1 à 8% en poids, et encore mieux de 2 à 5% en poids.

17. Composition selon l'une des revendications précédentes, **caractérisée en ce que** l'huile ayant un paramètre de solubilité δₐ compris entre 0 et 7,00 (J/cm³)^{1/2} est choisie parmi les huiles hydrocarbonées ou les huiles siliconées.

18. Composition selon l'une des revendications précédentes" **caractérisée en ce que** l'huile ayant un paramètre de solubilité δₐ compris entre 0 et 7,00 (J/cm³)^{½} est choisie parmi les alcanes aprotiques.

19. Composition selon la revendication précédente, **caractérisée en ce que** l'alcane aprotique présente une masse moléculaire allant de 165 à 900 g/mol, et plus préférentiellement de 250 à 800 g/mol.

20. Composition selon l'une des revendications 18 ou 19" **caractérisée en ce que** l'alcane aprotique est choisi parmi les polyisobutylène de poids moléculaire compris entre 250 et 800 g/mol, le squalane, l'huile de parléam et leurs mélanges.

21. Composition selon l'une des revendications 1 à 17, **caractérisée en ce que** l'huile siliconée ayant un paramètre de solubilité δₐ compris entre 0 et 7,00 (J/cm³)^{1/2} est choisies parmi les huiles de silicone linéaires telles que les polydiméthylsiloxanes, la cyclopentadiméthylsiloxane, la cyclotétradiméthylsiloxane, la phényl triméthicone, la cyclohexadiméthylesiloxane et leur mélange.

22. Composition selon la revendication précédente, **caractérisée en ce que** l'huile siliconée est ramifiée par des groupements de faible poids moléculaire, notamment en C₁-C₃, tel que des groupements méthyle ou éthyle.

23. Composition selon la revendication précédente, **caractérisée en ce que** l'huile siliconée ayant un paramètre de solubilité δₐ compris entre 0 et 7,00 (J/cm³)^{½} présente un poids moléculaire compris entre 100 et 800 g/mol, et plus préférentiellement de 300 à 500 g/mol.

24. Composition selon l'une quelconque des revendications précédentes, **caractérisée en ce que** l'huile ayant un paramètre de solubilité δₐ compris entre 0 et 7,00 (J/cm³)^{½} peut être présente dans la composition selon l'invention en une teneur allant de 20 à 100% en poids, par rapport au poids total de la phase grasse liquide continue, de préférence de 40 à 99% en poids, et mieux encore de 60 à 95% en poids.

25. Composition selon l'une quelconque des revendications précédentes, **caractérisée en ce qu'**elle comprend au moins un agent épaississant additionnel des huiles différent du composé de formule générale (I), choisi parmi les agents épaississants polymériques, les agents épaississants minéraux, et leurs mélanges.

26. Composition selon la revendication précédente, **caractérisée par le fait que** l'agent épaississant des huiles est présent dans la composition en une teneur totale allant de 0,1 % à 10 % en poids, par rapport au poids total de la composition, de préférence allant de 1 % à 7 % en poids, et préférentiellement allant de 2 % à 6 % en poids.

27. Composition selon l'une quelconque des revendications précédentes, **caractérisée par le fait qu'**elle comprend une phase aqueuse.

28. Composition selon l'une quelconque des revendications précédentes, **caractérisée par le fait qu'**elle comprend de l'eau et des huiles en une teneur telle que le rapport pondéral eau sur huile est supérieur ou égal à 1, et notamment compris entre 1 et 1,2.

29. Composition selon l'une quelconque des revendications précédentes, **caractérisée par le fait qu'**elle comprend une phase pulvérulente comprenant des pigments, des charges, des nacres et/ou leur mélange.

30. Composition selon l'une quelconque des revendications précédentes, **caractérisée par le fait qu'**elle comprend au moins une cire.

31. Composition selon l'une quelconque des revendications précédentes, **caractérisée par le fait qu'**elle comprend au moins un agent épaississant minéral.

32. Composition selon l'une quelconque des revendications précédentes, **caractérisée par le fait qu'**elle se présente sous forme anhydre, émulsions eau-dans-huile, huile-dans-eau ou multiples, et se présenter sous forme de gel, crèmes, suspensions, compact, coulés à chaud et sticks.

33. Procédé de maquillage ou de soin des matières kératiniques comprenant l'application sur lesdites matières kératiniques d'au moins une composition selon l'une quelconque des revendications précédentes.

34. Utilisation d'une composition selon l'une quelconque des revendications 1 à 32, pour obtenir un résultat maquillage lissant par une nivellement du relief de la peau et/ou mat et/ou doux au toucher.
